# EUROPEAN PATENT SPECIFICATION

(11) **EP 3 152 296 B1**
(45) Date of publication and mention of the grant of the patent: **04.08.2021**
(21) Application number: 15726971.3
(22) Date of filing: 05.06.2015
(51) Int. Cl.: C12N 5/071

(54) **HUMAN HEPATIC 3D CO-CULTURE MODEL AND USES THEREOF**
MENSCHLICHES HEPATISCHES 3D-CO-KULTIVIERUNGSMODELL UND VERWENDUNGEN DAVON
MODÈLE DE COCULTURE HÉPATIQUE HUMAINE TRIDIMENSIONNELLE ET SES UTILISATIONS

(30) Priority: 06.06.2014 GB 201410048; 25.03.2015 GB 201505012
(43) Date of publication of application: 12.04.2017
(73) Proprietor: Vrije Universiteit Brussel, 1050 Brussel (BE)
(72) Inventor: LEITE, Sofia Batista, B-1090 Brussel (BE); VAN GRUNSVEN, Leo, B-1090 Brussel (BE)
(74) Representative: Arnold & Siedsma
(86) International application number: PCT/EP2015/062551
(87) International publication number: WO 2015/185714

(56) References cited:
- WO-A1-02/48318
- US-A1- 2006 172 416
- SEUNG-A LEE ET AL: "Spheroid-based three-dimensional liver-on-a-chip to investigate hepatocyte-hepatic stellate cell interactions and flow effects", LAB ON A CHIP, vol. 13, no. 18, 1 January 2013 (2013-01-01), page 3529, XP055205695, ISSN: 1473-0197, DOI: 10.1039/c3lc50197c
- FONTANA L ET AL: "Ethanol induces the expression of alpha1(I) procollagen mRNA in a co-culture system containing a liver stellate cell-line and freshly isolated hepatocytes", BIOCHIMICA ET BIOPHYSICA ACTA. MOLECULAR BASIS OF DISEASE, AMSTERDAM, NL, vol. 1362, no. 2-3, 31 December 1997 (1997-12-31), pages 135-144, XP004276728, ISSN: 0925-4439, DOI: 10.1016/S0925-4439(97)00056-2
- PETRA KRAUSE ET AL: "Maintaining hepatocyte differentiation in vitro through co-culture with hepatic stellate cells", IN VITRO CELLULAR & DEVELOPMENTAL BIOLOGY - ANIMAL, vol. 45, no. 5-6, 28 January 2009 (2009-01-28), pages 205-212, XP055206223, ISSN: 1071-2690, DOI: 10.1007/s11626-008-9166-1
- SUSAN FUGETT ABU-ABSI ET AL: "Three-dimensional co-culture of hepatocytes and stellate cells", CYTOTECHNOLOGY, KLUWER ACADEMIC PUBLISHERS, DO, vol. 45, no. 3, 1 July 2004 (2004-07-01), pages 125-140, XP019236845, ISSN: 1573-0778, DOI: 10.1007/S10616-004-7996-6
- THOMAS ROBERT J ET AL: "The effect of three-dimensional co-culture of hepatocytes and hepatic stellate cells on key hepatocyte functions in vitro", CELLS TISSUES ORGANS, KARGER, BASEL, CH, vol. 181, no. 2, 1 January 2005 (2005-01-01), pages 67-79, XP009185643, ISSN: 1422-6405
- RICCALTON-BANKS LISA ET AL: "Long-term culture of functional liver tissue: Three-dimensional coculture of primary hepatocytes and stellate cells.", TISSUE ENGINEERING, vol. 9, no. 3, June 2003 (2003-06), pages 401-410, XP002743036, ISSN: 1076-3279
- LEITE S B ET AL: "P0439 : Human 3D hepatic co-culture model for in vitro drug-induced fibrosis testing", JOURNAL OF HEPATOLOGY, vol. 62, April 2015 (2015-04), XP029161108, ISSN: 0168-8278, DOI: 10.1016/S0168-8278(15)30648-6
- None

## Description

### FIELD OF THE INVENTION

The application in general relates to human hepatic 3D co-culture models, more in particular 3D spheroid co-cultures of human hepatocyte-like cells and hepatic stellate cells. Furthermore, the application provides a method for obtaining such co-cultures, as well as the use of said co-cultures in the identification of pro-fibrotic and/or anti-fibrotic compounds.

### BACKGROUND TO THE INVENTION

Chronic liver diseases, such as alcoholic liver disease, non-alcoholic fatty liver disease and viral hepatitis, lead to liver fibrosis and subsequent liver cirrhosis. According to WHO, liver cirrhosis accounts for 1.8% of all deaths in Europe, causing around 170.000 deaths/year, with a higher prevalence in east and west Europe. Approximately 1500 people/year die in Belgium as a result of chronic liver disease that has developed into cirrhosis. However, in terms of fibrosis, no pharmacological agent has been approved for routine use in a clinical context.

Hepatic Stellate Cells (HSC), have been identified as the key players in fibrosis. In the quiescent state, HSCs store vitamin A and have a balanced extracellular matrix (ECM), but upon (chronic) liver injury these cells transdifferentiate into myofibroblasts. Although *in vivo* HSC activation can be the result of direct activation of the HSCs, in the majority of the cases it is a response to hepatocyte injury and the subsequent interplay of different liver cells. During said transdifferentiation process the cells lose vitamin A, and show increased proliferation and motility, as well as increased ECM deposition, thereby causing liver fibrosis and subsequent cirrhosis of the liver.

Hence, there is a particular need for assays suitable for identifying pro-fibrotic and anti-fibrotic compounds, preferably thereby taking into account the indirect activation of HSCs caused by hepatic injury.

The HSC transdifferentiation process can be mimicked *in vitro* by plating the cells in regular culture dishes, partly due to the contact with a rigid surface, For example, Krause et al., 2009 (In vitro Cell. Dev. Biol. 45:205-212) provides a 2D co-culture of HSC with hepatocytes. However, in such co-cultures the activation process of HSCs cannot be controlled for the testing of compounds. On the contrary, *in vitro* strategies to keep the HSC quiescent, to allow controlled activation and the testing of anti-fibrotic and pro-fibrotic compounds, are still scarce. Hence, it was an object of the present application to provide cell culture systems and culture methods to keep quiescent-like HSCs in culture for long periods of time, and that allow the testing of potential pro-or anti-fibrotic compounds in a controlled fashion.

We have now surprisingly found that a 3D spheroid co-culture model of hepatocyte-like cells and HSCs, wherein the HSCs are present in equal or excess amounts of the hepatocyte-like cells, is a valuable model for testing potential pro-or anti-fibrotic compounds. In particular, we have found that in such co-cultures the hepatocyte-like cells keep their metabolic functionality and the HSCs are kept in a "quiescent" state for a longer period of time, thereby in particular allowing the analysis of pro-fibrotic compounds. When exposed to hepatotoxic compounds, even when biotransformation is necessary, such as APAP (Acetaminophen), the toxicity caused in the metabolically active hepatocyte-like cells induces activation in the surrounding HSCs. In contrast to the currently used 2D mono-cultures, wherein activation of the HSCs is already induced by contact with the culture plate surface (Mannaerts et al., 2010; Hepatology 51(2): 603-614), thereby often resulting in false positive results, in particular when testing pro-fibrotic compounds.

3D Mono-culture models of hepatocyte-like cells, more in particular HepaRG cells, and the use thereof for analyzing hepatotoxic activity of compounds such as APAP, have been described before (Leite et al., 2012; Toxicological Sciences 130(1), 106-116; Gunness et al., 2013; Toxicol. Sci 133(1), 67-78). However, this model is not suitable for analyzing pro-or anti-fibrotic activity of compounds, since the effect on the activation status of HSCs, the main fibrosis mediators, cannot be assessed. Liver diseases such as choleastasis and steatosis can be mimicked in *in vitro* cultures of hepatocytes by inspection of hepatocyte morphological changes. In the case of fibrosis, when the compound acts via hepatocytes (and not on HSC directly), the factors secreted or released by the hepatocytes are the ones that will activate (or not) HSCs, the latter being the criteria for a pro-fibrotic compound.

Also, 3D co-culture models including hepatocytes are known in the art. For example, Leite et al., 2011 (Toxicology in Vitro 25; 825-832) provides 3D hepatocyte-fibroblast co-cultures for toxicological applications, and addresses the effect of such co-cultures on hepatocyte functionality. However, it is silent on the functionality of the co-cultured fibroblasts, and it does not address the use of such models for pro-or anti-fibrotic compound testing. Thomas et al., 2005 (Cells Tissues Organs; 181: 67-79), and Thomas et al., 2006 (European Cells and Materials; Vol. 11 2006 pg.16-26) provide a 3D co-culture of hepatocytes and activated stellate cells cultured in a ratio of 1 (HSC) : 2 (Hepatocytes). Abu-Absi et al., 2004 provides a 3D co-culture of hepatocytes and activated stellate cells cultured in a ratio of 1 (HSC) : 10 (Hepatocytes). Riccalton-Banks et al., 2003 (Tissue Engineering; Vol. 9(3) pg. 401-410) provides a 3D co-culture of hepatocytes and activated stellate cells cultured in different ratios of 1 (HSC) : 10 (Hepatocytes), 1 (HSC) : 5 (Hepatocytes) and 1 (HSC) : 2 (Hepatocytes). Wong et al., 2011 (Biomaterials 32 8087-8096) provides a 3D co-culture of hepatocytes and stellate cells cultured in a ratio of 1 (HSC) : 3 (Hepatocytes), to mimick an activated HSC environment. However, all of these publications focus on hepatocyte functioning and none of them addresses the functionality of the co-cultured HSCs. More in particular, none of them addresses the relevance of the "quiescent" state of the HSC for pro-or anti-fibrotic compound testing, in contrast they support the use of pre-activated HSCs.

Hence, the prior art at hand does not provide a solution to the uncontrolled activation of HSCs associated with 2D mono-cultures, and in fact even supports the use of pre-activated cells. In contrast, we have surprisingly found that a 3D co-culture model of hepatocyte-like cells and HSCs, wherein the HSCs are present in equal or excess amounts of the hepatocyte-like cells, with inexistent cell attachment (free-floating), results in a stable culture of functional hepatocyte-like cells with only marginal activation of HSCs. Only upon simulation of drug induced liver injury (DILI) and especially incubation with pro-fibrotic compounds, indirect activation of the HSCs occurs via their close interaction with the hepatocyte-like cells. As discussed above, the prior art, teaches 3D co-cultures having excess amounts of hepatocytes, and does not teach or suggest to use excess amounts of HSCs let it be to address the effects of such ratio on the activation state of the HSCs.

### SUMMARY OF THE INVENTION

In a first aspect, the present application provides a 3D co-culture comprising hepatocyte-like cells and hepatic stellate cells (HSC); wherein said hepatic stellate cells are present in equal or excess amounts of said hepatocyte-like cells. In a particular embodiment, the amount of HSC cells is about double the amount of hepatocyte-like cells.

In a particular embodiment of the present application, the hepatocyte-like cells are human hepatocyte-like cells; in particular human hepatocyte-like cells selected from the list comprising: primary human hepatocytes, HepaRG cells, human embryonic stem cells (hESC) differentiated into hepatocyte-like cells, human induced pluripotent stem cells (hiPSC) differentiated into hepatocyte-like cells, primary fibroblast transdifferentiated into hepatocyte-like cells, and hepatocyte-like cell lines such as HepG2 or Upcyted hepatocytes; more in particular HepaRG cells.

In a further particular embodiment, the hepatic stellate cells of the current application, are human hepatic stellate cells; in particular human hepatic stellate cells selected from the list comprising: primary human hepatic stellate cells, human embryonic stem cells (hESC) differentiated into HSC-like cells, human induced pluripotent stem cells (hiPSC) differentiated into HSC-like cells, and human hepatic stellate cell lines such as LX-2, JS-1, hTERT-HSC, or upcyted-HSC; more in particular primary human hepatic stellate cells.

In the context of the present application, the 3D co-culture is preferably free-floating in the culture medium.

The present application further provides the use of the 3D co-culture according to this application in a screening assay for pro-fibrotic and/or anti-fibrotic compounds; more in particular for the screening of pro-fibrotic compounds.

In a further aspect, the present application provides a screening assay for pro-fibrotic and/or anti-fibrotic compounds; said assay comprising the steps of:
(a) providing a 3D co-culture according to this application,
(b) incubating said co-culture with a test compound, and
(c) determining whether said test compound induces or inhibits transdifferentiation and/or activation of said hepatic stellate cells;
wherein a compound that induces transdifferentiation and/or activation of said hepatic stellate cells is a pro-fibrotic compound; and wherein a compound that inhibits a fibrotic-compound induced-trans-differentiation and/or activation of said hepatic stellate cells is an anti-fibrotic compound.

In a particular embodiment, in step (c) of the screening assay according to this application, Col1a1, Col3a1 and/or Loxl2 expression levels may be used as a read-out for hepatic stellate cell activation or transdifferentiation. In said particular embodiment, a compound that increases Col1a1, Col3a1 and/or Loxl2 expression is considered a pro-fibrotic compound; and a compound that reduces Col1a1, Col3a1 and/or Loxl2 expression is considered an anti-fibrotic compound.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

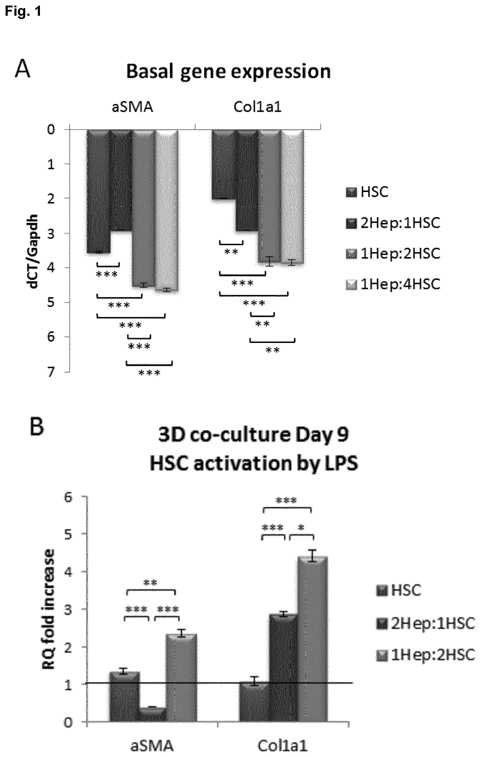

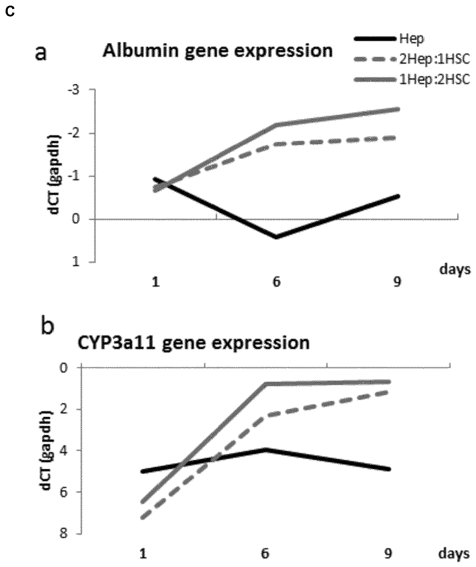

**Figure 1****:** *Screen of hepatocyte:hepatic stellate cell ratios in 3D mouse cultures.* Figure represents the HSC activation markers (**A, B**) and Hepatocyte markers (**C**) measured in the different 3D mouse hepatic co-cultures. **A** - basal gene expression of fibrotic markers (aSMA and Col1a1) on day 9 of culture. **B** - Fold increase of aSMA and Col1a1 on day 9 upon 6 days treatment with the pro-fibrotic factor LPS. Horizontal line corresponds to non-treated 3D co-cultures for each case. **C** - over-time gene expression of albumin (a) and CYP3A11 (b) in the 3D co-cultures where better results were obtained previously (2hep:1HSC and 1Hep:2HSC). Comparisons between groups were analyzed for statistical significance by one-way ANOVA followed by Tukey. *** p<0.001; ** p< 0.01; * p<0.05.

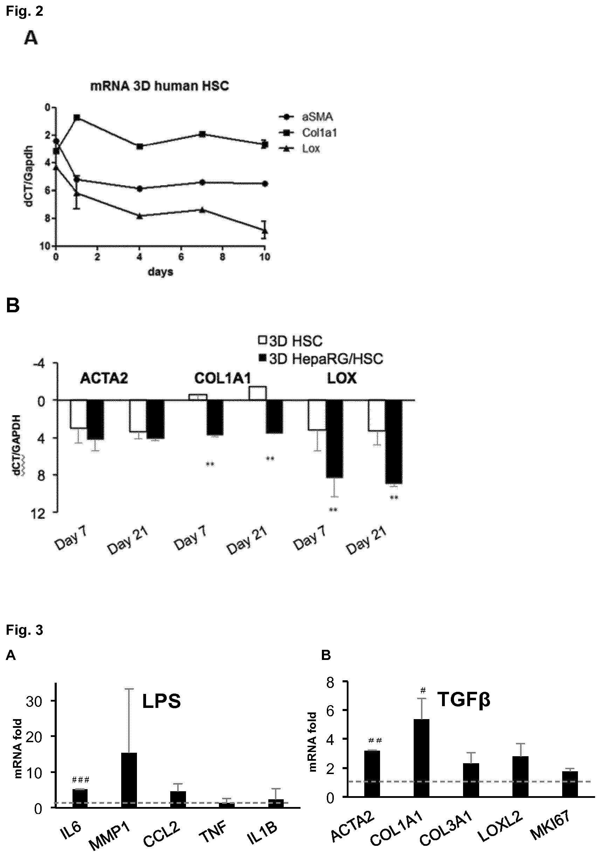

**Figure 2****:** *Human 3D HSC activation*/*inactivation gene expression.* **A** - gene expression of activation markers in 3D human HSC kept in 3D spheroids for 10 days in regular HSC medium. **B** - gene expression of activation markers in 3D Co-culture and respective 3D HSC control in HepaRG medium on days 7 and 21. Each point/bar represents a pool of 6 spheroids, error bars represent standard deviation values. *p<0,05 -3D HepaRG/HSC vs 3D HSC.
**Figure 3****:** *mRNA fold increase of HSC markers upon incubation with reference pro-fibrotic factors.* **A** - fold increase of 6 day LPS-treated cells (on first and third weeks of culture) over non treated cells. Values are means ± s.d. (n=6 spheroids). #p<0,05 -treated HepaRG/HSC vs non-treated 3D HepaRG/HSC. **B** - mRNA expression, of 3D HepaRG/HSC co-culture day 21, upon 48h exposure to 10 ng/ml TGFβ; fold increase from the solvent control.

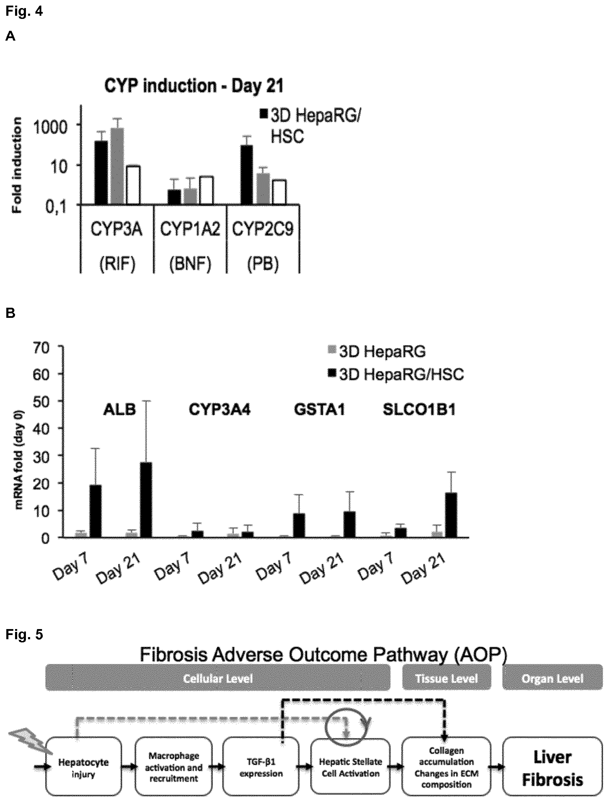

**Figure 4****:** *Hepatocyte profile in 3D HepaRG*/*HSC co-culture vs 3D HepaRG mono-culture.* **A** - CYP induction in the HepaRG cultures upon exposure to prototype CYP inducers (Rifampicin - RIF; β-Naphtoflavone - BNF; Phenobarbital - PB) for 48 hours on day 21; error bars represent the standard deviation between the 3 independent assays. **B** - Gene expression of reference hepatocyte markers (Albumin; Phase I - CYP 3A4); Phase II -GSTa1; transporter SLCO1B1 in the HepaRG cultures on days 7 and 21 of culture on the 3 independent assays. Error bars represent the standard deviation between 2 measurements.
**Figure 5****:** *3D HepaRG*/*HSC culture resume.* - Fibrosis Adverse Outcome Pathway

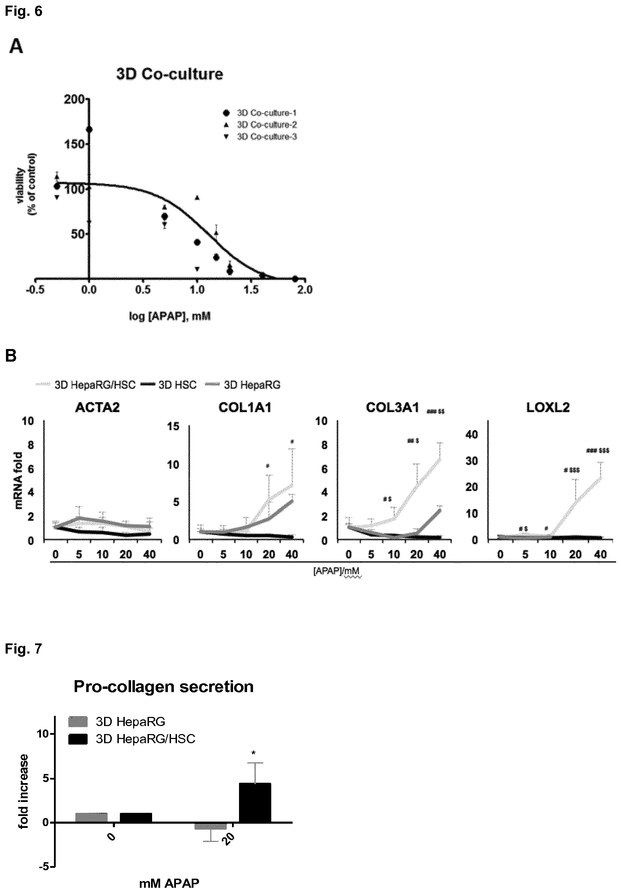

**Figure 6****:** *24h APAP dose-exposure on 3D Co-culture and respective mono-culture controls.* **A** - Cell viability in 3D co-culture after 24 hours incubation with different concentrations of APAP on day 21. The graph shows 3 independent experiments where each point represents N= 5 spheroids. Bars represent standard deviation. **B**- mRNA gene expression APAP dose-response activation in 3D Co-culture and respective controls. Each point represents the pool of 5 spheroids. Error bars are standard deviation of the PCR analysis. *p<0,05 vs 3D HepaRG/HSC culture, #p<0,05 vs solvent (OmM APAP) control.
**Figure 7****:** *Pro-collagen secretion. 3D HepaRG*/*HSC and respective mono-culture controls.* Procollagen measured in the suppernantant 24h after incubation with APAP/solvent in 3D HepaRG mono and 3D HepaRG/HSC co-culture. n= 3 assays (suppernatant pooled from 6 spheroids).

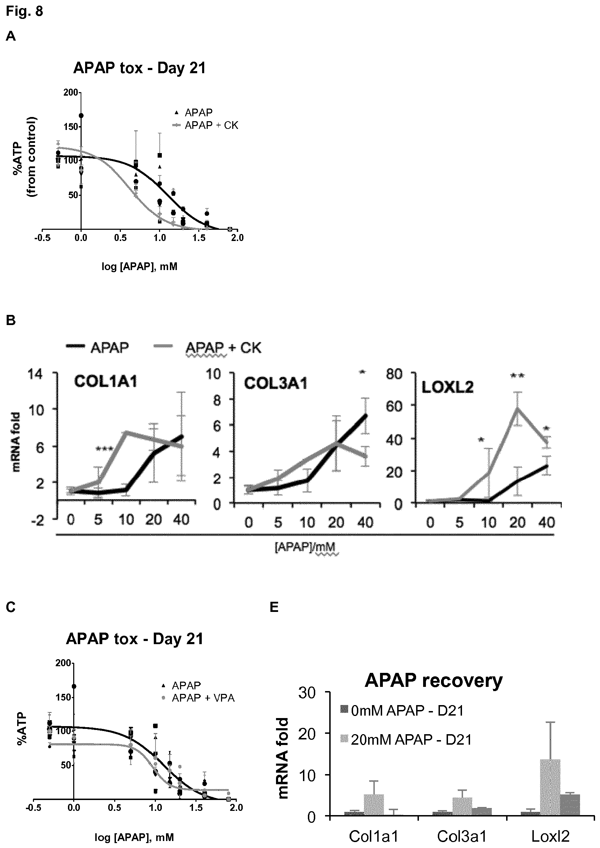

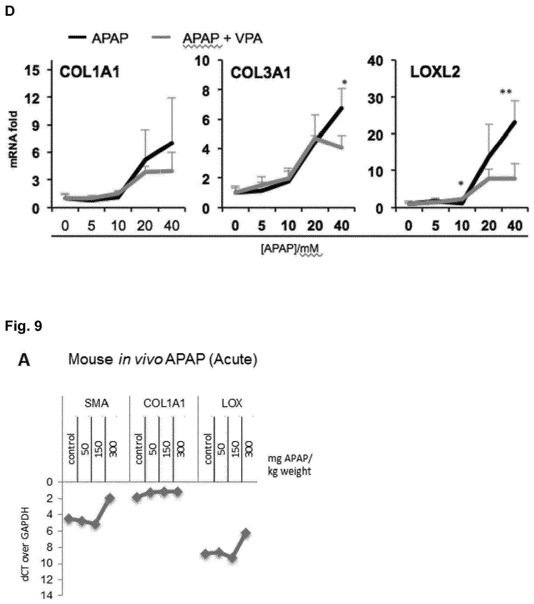

**Figure 8****:** *In vitro confirmation of the APAP in vitro data.* - Cell viability of 3D HepaRG/HSC co-culture exposed to APAP alone or together with (**A**) cytokine mixture (CK) or (**C**) fibrosis inhibitors such as Valproic Acid (VPA). Relative mRNA expression from the control (OmM APAP). Effect of the inflammatory mixture (**B**) and fibrosis inhibitors (**D**) and wash out and recovery on the activation markers (**E**). *p<0,05; test vs APAP alone n≥2 independent assays (4-6 spheroids/condition).

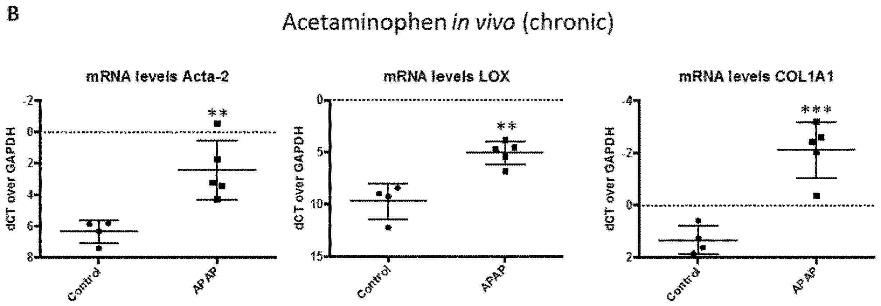

**Figure 9****:** *Hepatic stellate cell activation markers gene upon acute and chronic exposure to APAP.* **A** - SMA, Col1a1 and Lox dose-response gene expression on mouse HSC isolated after 24h of single dose injection of the named doses of APAP. **B** - gene expression of the same markers upon chronic exposure (4 weeks) to 375 mg APAP/kg weight. Statistical analysis using Unpaired T-test had significantly differences between treated (APAP) and control mice *** p<0.001; ** p< 0.01; * p<0.05.

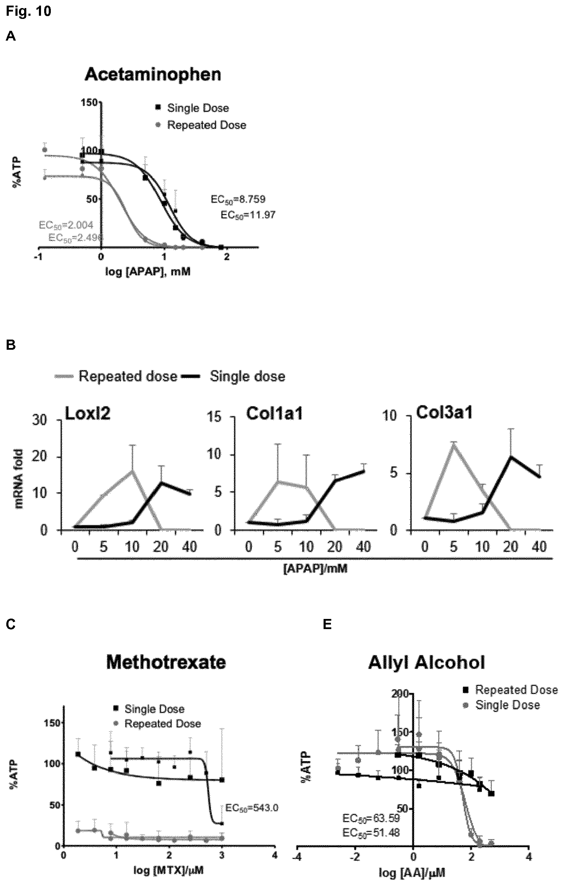

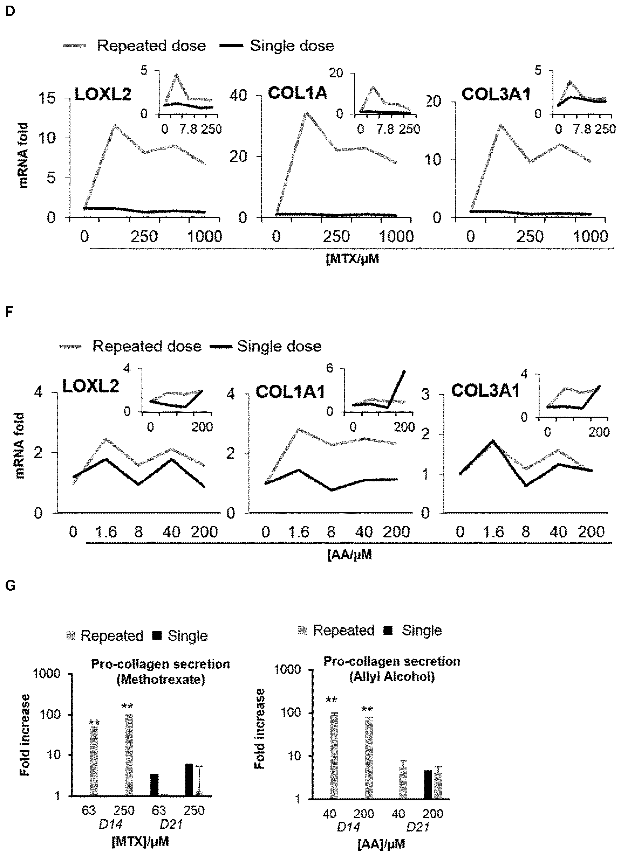

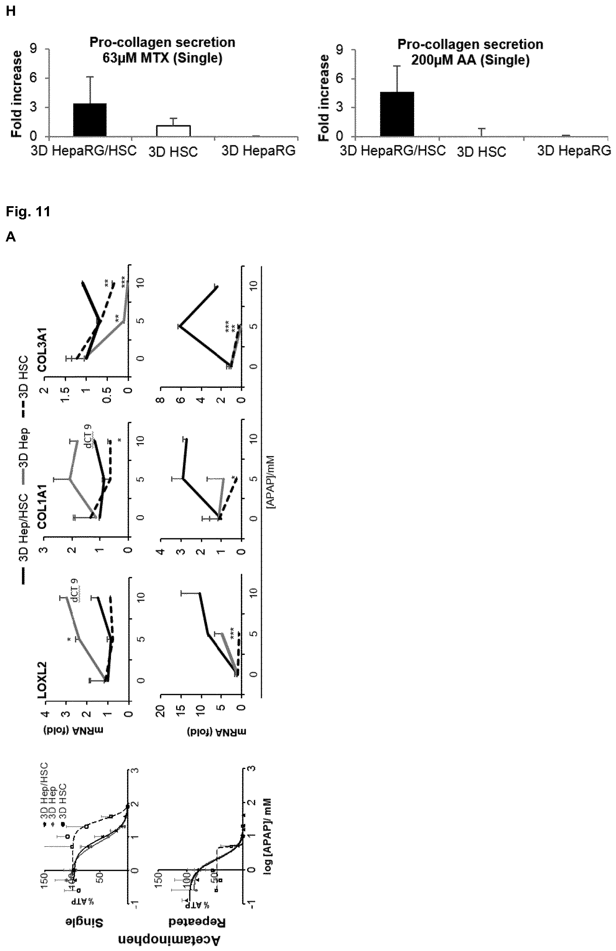

**Figure 10****:** *APAP and reference fibrotic compounds in single- and repeated-exposure of the 3D HepaRG*/*HSC co-culture.* (**A**, **C** and **E**) cell viability, represented by %ATP and (**B**, **D** and **F**) gene expression. Error bars represent the standard deviation between independent assays (N=2) each point represents the pool of 5-6 spheroids. (**D**, **F**) Graph inserts represent the second repeat of the assay. Due to the variation between assays values could not be averaged together. Each point represents the pool of 5-6 spheroids. (**G-H**) Fibrosis read-outs at the protein level. (**G**) Procollagen measurements in culture supernatant upon single and repeated compound exposure (day 14 and 21) to 3D HepaRG/HSCs. (**H**) Procollagen measurements in culture supernatant upon 24h incubation with the reference pro-fibrotic compounds of 3D HepaRG and 3D HSCmono-cultures.

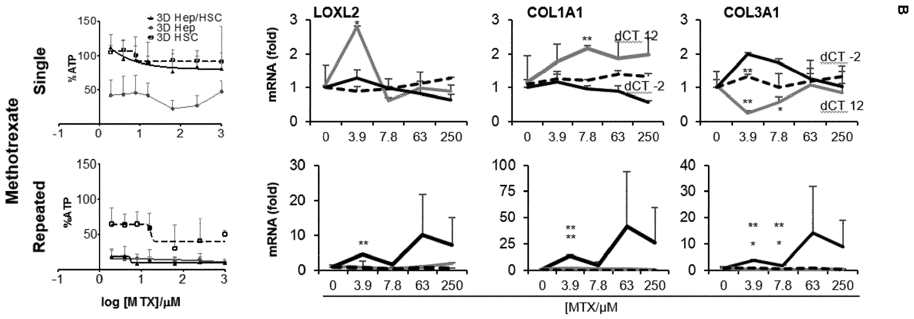

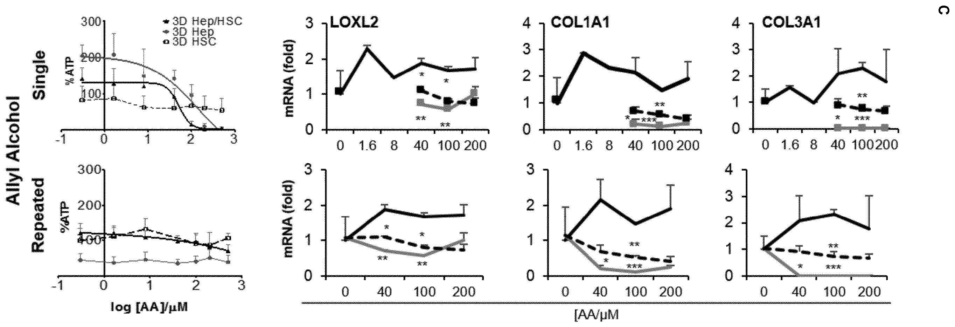

**Figure 11****:** *HSC recover after single and repeated exposure to Acetaminophen, Methotrexate and Allyl Alcohol.* - Activation markers 24h upon last exposure and 3 days washout.

### DETAILED DESCRIPTION OF THE INVENTION

As evident from the examples that follow hereinafter, we have found that a 3D co-culture comprising hepatocyte-like cells and equal or excess amounts of hepatic stellate cells (HSC), results in a stable culture of functional hepatocyte-like cells with only marginal activation of HSCs in the absence of stimulating compounds. It is only upon incubation with pro-fibrotic compounds, that direct or indirect activation of the HSCs, via their close interaction with the hepatocyte-like cells, occurs. Therefore, the 3D co-cultures according to this application, are preferred over the prior art known co-cultures (which include excess amounts of hepatocytes over HSCs), in particular for the testing of pro-fibrotic compounds.

Hence, in a first aspect, the present application provides a 3D co-culture comprising hepatocyte-like cells and hepatic stellate cells (HSC); wherein said hepatic stellate cells are present in equal or excess amounts of said hepatocyte-like cells. In a particular embodiment, the amount of HSC cells is about double the amount of hepatocyte-like cells.

In the context of the present application the term 'co-culture' is meant to be an *in vitro* cell culture system containing at least two distinct cell types, more in particular hepatocyte-like cells and (quiescent-like) hepatic stellate cells. Conversely, the term 'mono-culture' is meant to be a cell culture system containing only 1 type of hepatic cells. In a preferred embodiment, in the context of the present application, the co-culture is a three-dimensional (3D) co-culture of cells preferably in the form resembling a sphere (i.e. spheroid 3D co-culture).

The term 'hepatocyte-like cells' is meant to include cells which have similar functionalities as primary hepatocytes, and in particular show phenotypical features of functional hepatocytes when exposed to hepatogenic growth factors. Said phenotypical features may include expression and nuclear localization of liver-specific transcription factors, expression and polarization of drug transporters, expression of plasma proteins and typical building blocks of hepatic intercellular communication, activity of drug metabolizing enzymes at a level similar to primary hepatocytes. In particular, in the context of the present application, hepatocyte-like cells are meant to include primary human hepatocytes, HepaRG cells, human embryonic stem cells (hESC) differentiated into hepatocyte-like cells, human induced pluripotent stem cells (hiPSC) differentiated into hepatocyte-like cells, primary fibroblast transdifferentiated into hepatocyte-like cells, or any hepatocyte-like cell line such as HepG2 or Upcyted hepatocytes...; in particular HepaRG cells.

'Hepatic stellate cells (HSC)' also known as perisinusoidal cells or Ito cells are pericyte-like cells found in the perisinusoidal space (area between the sinusoids and hepatocytes) of the liver, also known as the space of Disse. In particular, in the context of the present application, hepatic stellate cells are meant to include human hepatic stellate cells; in particular human hepatic stellate cells selected from the list comprising: primary human hepatic stellate cells, human embryonic stem cells (hESC) differentiated into HSC-like cells, human induced pluripotent stem cells (hiPSC) differentiated into HSC-like cells, and human hepatic stellate cell lines such as LX-2, JS-1, hTERT-HSC,upcyted-HSCs...; in particular primary human hepatic stellate cells.

As already indicated herein before, any contact of the HSCs with the culture plates may already be sufficient to have them activated. Hence, and in order to keep the cells in a quiescent-like state for as long as possible, in a preferred embodiment, the 3D co-cultures are free-floating in the medium. This is preferably achieved by culturing them in cell-repellent culture dishes, more preferably with permanent rotation or stirring. Alternatively, low attachment cell culture plates either or not in combination with cell repellent agents such as PDMS (polydimethylsiloxaan), POLYHEMA (poly-2- hydroxyethyl methacrylate) or pluronic-type block copolymers (based on ethylene oxide and propylene oxide) may be used, again preferably with permanent rotation or stirring.

The 3D co-cultures of the present application are in particular suitable for testing compounds suspected of having pro-and/or anti-fibrotic activity. Hence, in a further aspect, the present application provides the use of the 3D co-culture according to this application in a screening assay for pro-fibrotic and/or anti-fibrotic compounds; more in particular for the screening of pro-fibrotic compounds.

In the context of the present application a 'pro-fibrotic compound' is meant to be any compound that acts directly on HSC or indirectly via activation or action in other cell types resulting in HSC activation. By HSC activation is meant the (1) increased production of collagen, procollagen, loxl or any other ECM constitutive that can lead to fibrosis; (2) proliferation of the HSC by increasing their cell number accompanied or not by HSC motility and (3) by the change of gene expression increasing activation markers such as aSMA, members of the collagen family (Col1a1, Col3a1, etc..) or members of the Lox or Loxl family (Loxl1, Loxl2, etc). In the context of the present application an 'anti-fibrotic compound' is meant to be any compound that can inhibit or reverse the activation process in the HSCs or inhibit the signaling leading to this activation, excluding hepato-protective agents.

The present application also provides a screening assay for pro-fibrotic and/or anti-fibrotic compounds; said assay comprising the steps of:
(a) providing a 3D co-culture according to this application,
(b) incubating said co-culture with a test compound, and
(c) determining whether said test compound induces or inhibits transdifferentiation and/or activation of said hepatic stellate cells;
wherein a compound that induces transdifferentiation and/or activation of said hepatic stellate cells is a pro-fibrotic compound; and wherein a compound that inhibits a fibrotic-compound induced-trans-differentiation and/or activation of said hepatic stellate cells is an anti-fibrotic compound.

In a particular embodiment, in step (c) of the screening assay according to this application, Col1a1, Col3a1 and/or Loxl2 expression levels may be used as a read-out for hepatic stellate cell activation or transdifferentiation. In said particular embodiment, a compound that increases Col1a1, Col3a1 and/or Loxl2 expression is considered a pro-fibrotic compound; and a compound that reduces Col1a1, Col3a1 and/or Loxl2 expression is considered an anti-fibrotic compound.

The 3D co-culture of the present application may be obtained using the following general procedure:
- HSCs and hepatocyte-like cells were seeded in HepaRG thawing medium (William's Medium E supplemented with 2mM I-glutamine, 1% (v/v) pen/strep 5 µg/ml bovine insulin and 50µM hydrocortisone and 10% (v/v) FBS and 0.5% (v/v) DMSO). in cell-repellent culture plates; wherein the HSCs are added in equal or excess amounts of the hepatocyte-like cells
- The plates were kept in the cell culture incubator (Heracell 150, Thermo electron corporation) 37°C, 5%CO₂ for 10-15 min
- Within the cell culture incubator, the plates were stirred at 80 rpm during the entire period of culture
- After 24h, HepaRG culture medium without DMSO was added
- The medium was refreshed every 2 days by changing 90% of the medium with fresh HepaRG culture medium without DMSO

### EXAMPLES

### EXAMPLE 1: Selection of 3D co-culture ratios

### MATERIAL AND METHODS

### Liver cell isolations and seeding

Male balbC mice were used for all experiments; animals were housed in a controlled environment with free access to chow and water. Cells were isolated from mice aged 20-25 weeks after anesthesia with Nembutal. Previously described procedures for hepatic stellate cells (HSC) (Guimaraes et al., 2010; J. Hepatol 52(3): 389-397) and hepatocytes (Hep) (Goncalves et al., 2007 Malar J 6:169) were followed. Cells were maintained in hepatocyte culture medium consisting of William' E medium (1x) with 2mM of Gln, 20mU/ml insulin, 50nM dexamethasone, 2.5µg/ml fungizone, 50µg/ml gentamycin, 100µg/ml vancomycin, 100U/ml penicillin, 100µg/ml streptamycin, 7ng/ml glucagon and 10% FBS. After isolation, the different cell types were put together at different ratios (2Hep:1HSC; 1Hep:2HSC; 1Hep:4HSC) and seeded at 10 µl/well with a final amount of 5000 cells/well. Cells were kept in a low attachment plates with U bottom (Greiner 650185). 24h after seeding, 100 µl of the previously described medium without FBS was added to each well. Medium was refreshed every 2 days by changing 90% of the medium with medium lacking FBS.

### RNA analysis

Total RNA was extracted from cells using the ReliaPrep RNA Cell Miniprep System (Promega, Madison, WI). Reverse-transcription using the Revert Aid Kit (ThermoFisher Scientific, St. Leon-Rot, Germany) allowed conversion of RNA to cDNA. The RT reaction was performed at 25 °C for ten minutes followed by 30 minutes at 50 °C. For quantitative real-time polymerase chain reaction (qPCR), GoTaq QPCR Master Mix with BRYTE green (Promega, Madison, WI) was used, subjected to qPCR in a 7500 real time PCR system and analysed using System SDS software v2.0.5 (Applied Biosystems), using GAPDH for normalisation. Reliability of GAPDH as house-keeping gene was determined using Normfinder (Andersen, Jensen et al. 2004) to produce the ΔCT values. Besides ΔCT, mRNA gene expression results are also expressed in fold change differences between samples were determined using the comparative Ct (δδCt) method. The expression level of different targets, relative to GAPDH and relative to the calibrator, was given by2^{-δδCt}. Primers were produced by Integrated DNA Technologies (Leuven, Belgium).

### Albumin measurements

Albumin measurements were performed using the ELISA kit from AssayPro - Gentaur, following the manufacture instructions.

### RESULTS

The readouts used to decide on the best ratio of cells were to assess good quality and functionality of the cells: i.e. quiescent HSCs for longer times however still able to respond to pro-fibrotic factors and functional hepatocytes.

As evident from figure 1A, all co-culture ratios (2Hep:1HSC, 1Hep:2HSC, 1Hep:4HSC) could keep the HSC activation markers aSMA and Col1a1 at the same or lower level compared to the 3D HSC without hepatocytes. However, the best results (lower aSMA and Col1a1 expression) were achieved with the ratios of 1Hep:2HSC and 1Hep:4HSC (Fig.1A). Based on these results, further analysis of the 3D co-cultures was performed using the ratio of 1Hep:2HSC.

When stimulated with a pro-fibrotic factor (LPS) the 1Hep:2HSC ratio showed a significantly better activation response (increased aSMA and Col1a1), when compared to the 2Hep:1HSC ratio and an even greater activation response when compared to the 3D HSC without hepatocytes (Fig. 1B).

Finally, long term expression of the hepatocyte markers albumin and cyp3a11 (Fig.1C) was analyzed. The verification of an higher expression of the named markers in the 1Hep:2HSC 3D co-culture confirm the preference of higher amounts of HSCs in 3D co-culture for the testing of pro-fibrotic compounds.

### EXAMPLE 2: Characterization and proof testing of the 3D HepaRG/HSC co-culture

### MATERIAL AND METHODS

### HSC cells

Human cells Hepatic Stellate Cells (HSC) were isolated from hepatic non-parenchymal fraction and prepared for culture as described in Thoen et al., 2011 (J Hepatol 55(6): 1353-1360).

### HepaRG

Cells were obtained from Biopredic, 8M differentiated cells/vial and thawed at the day 0 (starting of the co-culture). Thawing was performed following the instructions of the providers.

### Medium

Cells were maintained in HepaRG maintenance medium without DMSO. All compound incubations (CYP inducers and APAP) were performed in Induction/Toxicity medium which is the same medium but without serum.

### Generation and maintenance of 3D cell spheroids

For the generation of the cell spheroids, 96-well plates treated with cell- repellent (Greiner ref. 650970) are used. HepaRG cells are directly used upon thawing and HSCs were trypsinized and collected in HepaRG thawing medium. Mono- and co-culture suspensions were prepared in HepaRG thawing medium as shown below and seeded at 10µl/well:

**Table 1: 3D cell seeding**

| *Type of culture* | *HSC seeding* | *HepaRG seeding* |
|---|---|---|
| 3D HepaRG mono-culture | - | 0.2 million cells/ml |
| 3D HSC mono-culture | 0.2 million cells/ml | - |
| 3D HepaRG/HSC co-culture | 0.133 million cells/ml | 0.067 million cells/ml |

After 10-15min in the incubator, plates were stirred at 80 rpm for the entire period of the culture. 24h after seeding, 100 µl of HepaRG culture medium 0% DMSO was added to each well. Medium was refreshed every 2 days by changing 90% of the medium. 3D HepaRG/HSC co-culture and control mono-culture were cultured for 21 days. Cross diameter of the spheroids never exceeded 200 µm (date not shown).

### CYP induction

CYP induction assays were performed in 3D HepaRG/co-culture as well as in 3D HepaRG monoculture. For 48h, starting at day 19, cells were exposed to prototype inducers (530µM phenobarbital [PB], 10µM rifampicin [RIF], and 25µM β-naphthoflavone [BNF]), as previously described in Leite et al., 2012 (Toxicol Sci 130(1): 106-116) using HepaRG induction/toxicity medium. Controls were kept in the same medium with the same amount of solvent (0.1%DMSO). Spheroids were exposed to the inducers individually. After the incubation time, cells were washed and pulled, 5 spheroids/condition to perform the activity assay using the P450-Glo™ CYP2C9 Assay, CYP3A and CYP 1A2 (Promega) respectively, following the instructions of the manufacturer. At the end of the incubation, results were normalized by total ATP content measured using the Cell titer Glo (Promega).

### Exposure to pro-fibrotic factors

From day 14 to day 21, 3D HepaRG/HSC spheroids were exposed to a solution of 100ng/ml Lipopolysaccharide (LPS, Sigma) in culture medium, refreshed every second day up to day 21. Incubation with the Transforming growth factor-β1 (TGF-β1, R&D systems inc., Minneapolis, MN, USA) was performed on day 19 for 48h at a concentration of 10 ng/ml in HepaRG Induction/Toxicity medium. On day 21, cells were lysed and gene expression was analyzed upon RNA extraction.

### APAP incubation

APAP toxicity assay was performed in 3D HepaRG/co-culture as well as in 3D HepaRG monoculture. For 24h, starting at day 20, cells were exposed to a range of APAP concentrations between 0.5 and 80 mM, keeping cells with the solvent (0.1%DMSO) as a control. For some experiments the culture medium was supplemented with 2.5 mM valproic acid (VPA sodium salt, Sigma-Aldrich) or with cytokine mixture (Raicevic et al.,2012) - interleukin (IL)-1β 25 ng/mL (Peprotech, Rocky Hill, NJ, USA), interferon (IFN)-γ 10³ U/mL, tumor necrosis factor (TNF)-α 50 ng/mL, and IFN-α 3 · 10³ U/mL (all from Prospec, Inc., East Brunswick, NJ, USA). For the recovery assay, at the end of APAP incubation, cells were washed using HepaRG culture 0% DMSO medium and kept in the same medium for 3 more days.

At the end of the incubation period, 5 spheroids per condition were pooled and lysed for RNA analyses, while the remaining spheroids per condition were lysed individually for viability analysis using the Cell titer Glo kit (Promega) for ATP measurements following the instructions of the manufacturer.

### RNA analysis

Total RNA was extracted from cells using the ReliaPrep RNA Cell Miniprep System (Promega, Madison, WI). Reverse-transcription using the Revert Aid Kit (ThermoFisher Scientific, St. Leon-Rot, Germany) allowed conversion of RNA to cDNA. The RT reaction was performed at 25 °C for ten minutes followed by 30 minutes at 50 °C. For quantitative real-time polymerase chain reaction (qPCR), GoTaq QPCR Master Mix with BRYTE green (Promega, Madison, WI) was used, subjected to qPCR in a 7500 real time PCR system and analysed using System SDS software v2.0.5 (Applied Biosystems), using GAPDH for normalisation. Primers were produced by Integrated DNA Technologies (Leuven, Belgium) are listed in Table 2.

**Table 2: Primers used for QPCR analysis (*mouse primers)**

| ***Gene*** | ***GenBank Acc N°*** | ***Forward Primer*** | ***SEQ ID N°*** | ***Reverse Primer*** | ***SEQ ID N°*** |
|---|---|---|---|---|---|
| GAPDH | NM_002046.4 | AGCCACATCGCTCAG ACAC | 1 | GCCCAATACGACCAAA TCC | 17 |
| ACTA 2 | NM_001141945.1 | CTGTTCCAGCCATCC TTCAT | 2 | TCATGATGCTGTTGTA GGTGG | 18 |
| COL1A1 | NM_000088.3 | GACACAGAGGTTTCA GTGG | 3 | CACCCTTAGCACCAAC AG | 19 |
| LOX | NM_001178102.1 | CACAGTTGTCATCAA CATTACC | 4 | CTGACCTTTAGGATAT AGTTTCCAG | 20 |
| IL6 | NM_000600.3 | GATGAGTACAAAAGT CCTGATCCA | 5 | CTGCAGCCACTGGTTC TGT | 21 |
| MMP1 | NM_001145938.1 | GCTAACCTTTGATGC TATAACTACGA | 6 | TTTGTGCGCATGTAGA ATCTG | 22 |
| | NM_002421.3 | | | | |
| CCL2 | NM_002982.3 | AGTCTCTGCCGCCCT TCT | 7 | GTGACTGGGGCATTGA TTG | 23 |
| TNF | NM_000594 | CCTCTCTCTAATCAG CCCTCTG | 8 | GAGGACCTGGGAGTA GATGAG | 24 |
| IL1B | NM_000576 | AGCTACGAATCTCCG ACCAC | 9 | CGTTATCCCATGTGTC GAAGAA | 25 |
| COL3A1 | NM_000090.3 | GGAGCTGGCTACTTC TCGC | 10 | GGGAACATCCTCCTTC AACAG | 26 |
| LOXL2 | NM_002318.2 | GGAGAGGACATACA ATACCAAAGTG | 11 | CCATGGAGAATGGCCA GTAG | 27 |
| MKI67 | NM_002417 | ACGCCTGGTTACTAT CAAAAGG | 12 | CAGACCCATTTACTTG TGTTGGA | 28 |
| ALB | NM_000477.5 | TGGCACAATGAAGTG GGTAA | 13 | CTGAGCAAAGGCAATC AACA | 29 |
| CYP3A4 | NM_017460.5 | CCTCCCTGAAAGATT CAGC | 14 | TGAAGAAGTCCTCCTA AGCT | 30 |
| | NM_001202855.2 | | | | |
| GSTA1 | NM_145740 | CTGCCCGTATGTCCA CCTG | 15 | AGCTCCTCGACGTAGT AGAGA | 31 |
| SLCO1B 1 | NM_006446.4 | GCAATAGCAATGATT GGTCC | 16 | CCAACCCATCGAGAAT CAG | 32 |

### Albumin measurements

Albumin measurments were performed using the ELISA kit from AssayPro - Gentaur, following the manufacture instructions.

### Pro-collagen measurements

Samples for pro-collagen measurement were five times concentrated using 10 kD Vivaspin 500 ultrafiltration columns (VWR, Radnor, Pennsylvania), the resulting supernatant was analysed for the presence of Type I C-Peptide using the TAKARA (Chanteloup-en-Brie, France) kit.

### Seahorse

Mitochondrial functions of the 3D HSC, 3D HepaRG and 3D HepaRG/HSC were explored using a XFe96 Analyser. Measurements of the Oxygen Consumption Rate (OCR) and Glycolysis (through proton production - Extracellular Acidification rate, ECAR) were done first in the basal conditions and upon APAP exposure. In the basal state, cells were challenged to stress situations using the XF Cell Mito Stress Test Kit (Seahorse Biosciences) following the instructions of the manufacturer. Equivalent assay was performed in the three culture systems after 12 and 24 hours of APAP incubation.

### Statistics

Comparisons between more than two groups were analyzed for statistical significance by one-way ANOVA followed by Tukey. Statistical analysis of values for comparison between two groups was performed using an independent sample t-test. Results were considered significant when p <0.05.

### RESULTS

For the development of the human 3D hepatic co-culture cryopreserved differentiated HepaRG were used as functional hepatocyte-like cells and human HSCs generated in-house were used as hepatic stellate. HSCs were isolated from the non-parenchymal fraction of human livers, expanded in culture and frozen as previously described and characterized by us (Thoen, Guimaraes et al. 2011). To generate the co-culture cell spheroids on day 0, specific cell amounts in single cell suspension from both cell types were put in contact with each other in non-attaching round wells and orbital stirring. This strategy allowed spontaneous and quick generation of spheroids and together with the fact that the cells do not expand in these conditions, we could ensure an optimal spheroid diameter at 200 *µ*m maximum for the entire culture time. It is known, that this size avoids the formation of necrotic cores mimicking also the maximum distance that a cell is from a blood vessel within the body. This size together with the active mass transfer induced by the stirring, allows a good nutrient and oxygen diffusion within the spheroid. Both control mono-cultures of HSC and HepaRG were kept with equivalent total cell amount/spheroid to ensure similar diffusion. Notably, after 21 days in culture, the cells in 3D HepaRG/HSC spheroid show a segregated organization with a concentration of HSCs in the core of the spheroid, while hepatocyte-like cells, the first target of compounds, accumulate more in the periphery (data not shown). Cell ratio and medium composition was optimized based on the better establishment of hepatocyte functions and less activated state of the HSCs. We achieved this balance by culturing the cells in a ratio of 1HepaRG:2HSC in a hepatocyte-based medium. As detailed below, we have found that by culturing HSC in 3D spheroids, the expression of regular fibrotic markers such as aSMA, Col1a1 and Lox, that are normally increased in 2D monolayer cultures, can be reduced (Fig.2A). As detailed in example 1, and confirmed herein below, this effect is further improved when HSCs are kept in equal or excess amounts in 3D co-culture spheroids with hepatocyte-like cells (HepaRG), in hepatocyte medium, (Fig.2B). Consequently, in order to test the HSCs functions at this stage, we analyzed the responsiveness of the 3D HepaRG/HSC to LPS and TGFβ, two known pro-fibrogenic factors that induce specific HSC responses. We observed that as it happens during liver fibrosis, LPS induced an up-regulation of the survivor markers (IL6, MMP1, CCL2, TNFα, IL1β) (Fig. 3A) while TGFβ acted on the activation markers (Acta2, Col1a1, Col3a1, Loxl2) (Fig. 3B). Such up-regulations could not be observed in the respective 3D mono-cultures (data not shown).

In spite of the inactive state of the HSCs in 3D mono-culture, when compared with regular 2D HSC cultures, little or inexistent activation was observed when challenged with pro-fibrotic factors. It is our believe that, as it happens *in vivo,* cells need multiple stimuli for activation, which in 2D is overcompensated by the rigidity of the tissue culture dish surface. In order to analyze the activation of HSCs in a controlled and more reliable way, we developed a more complex 3D system by introducing human hepatocyte-like cells (HepaRG (BioPredic, France), which are the main targets of most drugs and the main liver metabolizers. Notwithstanding of being insufficient as mono-culture to mimic a fibrotic response, the role of hepatocytes in this model is crucial not only as a trigger of HSC once injured, but also for the metabolization/biotransformation of drugs that can alter their effect in the development of liver fibrosis. There remains the need to ensure their functionalities, but also to avoid HSC activation in response to hepatocyte de-differentiation or death after a while in culture.
Our studies showed that the metabolic profile of the HepaRGs is not affected by the presence of HSCs (CYP-activity and inducibility - Fig. 4A; hepatocyte gene expression - Fig. 4B), and that the inactivation state of the HSCs is even improved (Fig. 2B) by the co-culture environment as mentioned before. Likewise, induction values are in alignment with CYP inducing capacities of HepaRG maintained in similar conditions (Table 3).

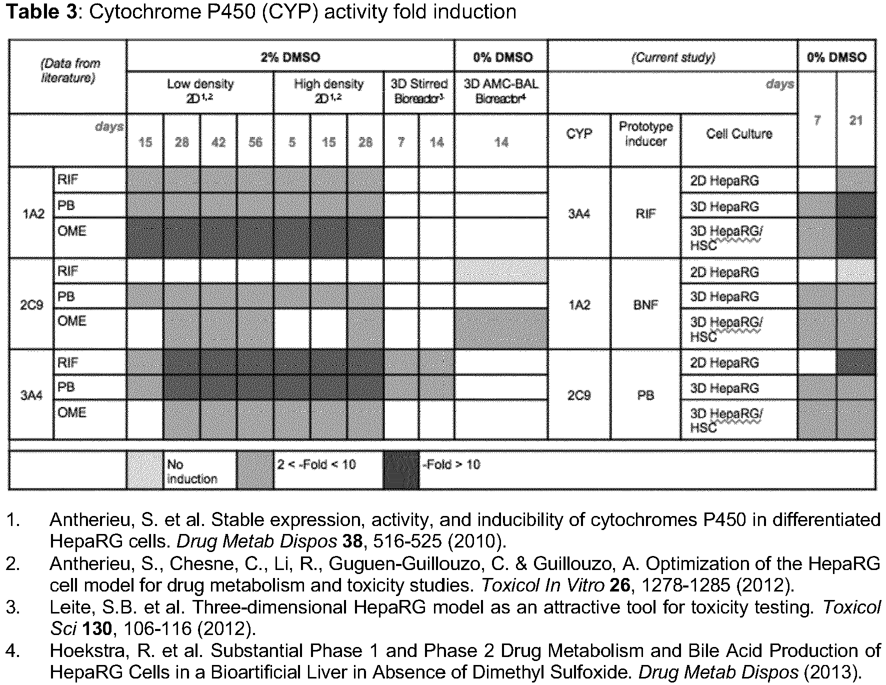

Additionally, CYP3A4 staining in the 3D HepaRG/HSC spheroid section, showed good protein expression (data not shown) since only 1/6 of the 3D co-culture spheroid cells (half of HepaRG) are hepatocyte-like cells. To complement the characterization of hepatocyte functions, we evaluated the transport capacities by the excretion of CMFDA, a MRP2 substrate. Once inside the cells, CMFDA is metabolized into its fluorescence form and then excreted by the functional hepatocytes. The visualization of its accumulation upon excretion, on specific sites resembling bile before totally wash out, was only possible upon fixation showing a good elimination capacity of the spheroid (data not shown).

In spite of the good metabolic activity in the first week (data not shown), values were more stable at the last week of culture, where the cell-cell interaction has been better promoted between the different cell types. Therefore, our further assays were design to have the cellular readouts at day 21.

After optimization of the strategy to efficiently generate 3D co-culture spheroids, with the proper cell ratio and correct media formulation for 21 days in culture, the model was validated for the use of a compound that would induce *in vitro* hepatocyte injury and consequent HSC activation. In order to exploit the capacity of our method to respond to fibrosis, we followed a fibrosis Adversed Outcome Pathway (AOP) developed by us and others (Fig. 5).

Acetaminophen (APAP) was used as a reference compound with a known outcome, i.e. hepatotoxicity and perhaps the ability to induce indirectly HSC activation. This also serves as a quality control of the hepatocyte/HSC co-culture since it verifies effective metabolisation by hepatocytes (cells die from the accumulation of NAPQI, a CYP2E1-mediated metabolite of APAP and this can indicate the good quality of the cells after 21 days) and the potential of the HSCs to activate upon hepatocyte injury. When the EC50 values (concentration at which the compound kills half of the population - Table 3) were evaluated, we confirmed that it is slightly decreased in the co-cultures when compared with the HepaRG mono-cultures (table 4). Since for APAP to become actively hepatotoxic it is necessary to be metabolized, the reduction most likely indicates that the CYP activity in the 3D co-cultures is higher than in the HepaRG monocultures. Furthermore, when compared with similar studies we can see that this activity is improving over time in our studies while in Gunness et. al. studies (Gunness et al., 2013; Toxicol. Sci 133(1), 67-78) this is decreasing. Besides the presence of the HSCs, we believe that this result is probably due to the absence of DMSO at the beginning and continuation of the culture.

**Table 4: APAP EC₅₀ values (± stdev)**

| | | **Day 6/7** | **Day 21/22** |
|---|---|---|---|
| ***Current study*** - 0% DMSO | **3D HepaRG** | **11,6** | **11,6 ± 2,15** |
| | **3D HepaRG/HSC** | **10,0 ±1,09** | **8,0 ± 4,17** |
| ***Gunness et al., 2013*** - 2% DMSO | **3D HepaRG** | **2,7 ± 7,14** | **10,1 ± 9,90** |

| | | | |
|---|---|---|---|
| (Gunness et al., 2013; Toxicol. Sci 133(1), 67-78) | | | |

Despite the toxic effect of APAP on the HSC, this is only observed at the highest concentrations (data not shown) normally not related with the CYP metabolization into NAPQI but mithocondrial oxidative stress. We investigated the effect of APAP on the different cell types by measurement of the two major energy producing pathways assessing at the mitochondrial functional level. For that purpose, we measured the Oxygen Consumption Rate (OCR) and Glycolisis (through proton production - Extracellular Acidification rate, ECAR) using the XF Cell Mito Stress Test Kit (Seahorse Biosciences) 0, 12 and 24 hours of APAP incubation in the three 3D cultures. When we compare the two 3D mono-culture, (OmM APAP) we detect a severely higher ATP production in the HepaRG, leading us to assume that majority of the ATP levels in the co-culture accrues from these cells (data not shown). We can then infer that the observed drop on the ATP levels in the 3D HepaRG/HSC, after APAP incubation is mainly a consequence of toxicity in HepaRG, most probably hepatotoxicity. The same assay allowed the comparison of the three culture systems regarding the recover from mithocondrial stress. When challenged in sequential respiratory inhibition and rescue, using Oligomycin, FCCP and Antimycin A + Rotenone, the higher OCR and ECAR values show that, both in basal and APAP conditions, the 3D HepaRG/HSC has better capacity to cope and recover from stress that any of the mono-cultures (data not shown). This effect difference is more pronounced when compared with the 3D HSCs. Additionally, the hepatotoxic effect of APAP is also confirmed by caspase 3 stainings concentrated in the remaining cells of the outer layer of the spheroid.
Additionally, results show that upon an efficient dose-response APAP toxicity (Fig. 6A), mRNA levels of several pro-fibrotic markers are induced in a dose-dependent fashion (Fig. 6B). When analyzing the relative improvement of expression of the activation markers Col1a1, Col3a1 and Loxl2 it can be confirmed that expression is only increased or in a higher extent in the co-culture (bold values in table 5) and not in the correspondent mono-cultures.

**Table 5: mRNA fold increase after exposure to 20 mM APAP (24h)**

| | | **3D HepaRG/HSC** | **3D HSC** | **3D HepaRG** |
|---|---|---|---|---|
| **aSMA** | ***Assay 1*** | **3,27 +/-0,12** | 0,67 +/-0,87 | *ND* |
| | ***Assay 2*** | 0,59 +/-0,03 | 0,38 +/-0,00 | 0,69 +/-0,09 |
| | ***Assay 3*** | 0,86 +/-0,10 | 0,17 +/-0,17 | **1,06 +/-0,13** |
| **Col1a1** | ***Assay 1*** | **51,73 +/-4,10** | ***ND*** | ***ND*** |
| | ***Assay 2*** | **3,68 +/-0,64** | 0,78 +/-0,03 | **3,01 +/-0,06** |
| | ***Assay 3*** | **10,21 +/-1,75** | 0,27 +/-0,27 | **4,80 +/-4,83** |
| **Col3a1** | ***Assay 1*** | **4,87 +/-0,01** | 0,37 +/-0,02 | ND |
| | ***Assay 2*** | **2,08 +/-0,10** | 0,08 +/-0,01 | **13,59 +/-14,64** |
| | ***Assay 3*** | **4,44 +/-0,20** | 0,16 +/-0,16 | 0,65 +/-0,02 |
| **Loxl2** | ***Assay 1*** | **302,54 +/-1,60** | **1,13 +/-0,05** | ***ND*** |
| | ***Assay 2*** | **9,49 +/-1,93** | 0,70 +/-0,23 | ***ND*** |
| | ***Assay 3*** | **22,97 +'-0,07** | 0,85 +/-0,86 | ***ND*** |

The irresponsiveness of ACTA2 in these conditions, together with parallel studies involving TGFβ-blockers (data not shown) made us doubt on the role of TGFβ HSC activation upon APAP. Yet, we could detect an increase at the protein level, in the center of the spheroid (data not shown). Regarding the collagen's fold increase, not much difference was detected between the 3D HepaRG/HSC and the 3D HepaRG, nevertheless, there is a 10 to 100x difference in the basal gene expression levels lower in the 3D HepaRG (data not shown). This differences in levels are translated into the amount of collagen secreted into the medium. Following the clinical method of measurement of the C-terminal pro-collagen in the cultures supernatants, we observed a significant increase of collagen secretion in the 3D HepaRG/HSC cultures upon APAP incubation, which is not observed in the 3D HepaRG cultures (Fig. 7). Additionally, we could further confirm collagen secretion, by staining and detection of cross-linked collagen (picrosirius) in the paraffin section of the 3D HepaRG/HSC (data not shown). Even though the collagen accumulation in the spheroid is quite reserved when compared with normal fibrotic liver biopsies, it is important to bear in mind that this is the result of a short insult.

### Confirmation of the APAP in vitro data

Acute liver failure as a consequence of exposure to high doses of APAP has been widely reported in animals and humans. However, the liver effects upon exposure to sub-lethal doses of APAP were never investigated, especially regarding fibrosis, where the foremost manner of *in vivo* HSC activation is hepatocyte injury. In order to warrant that the APAP-dependent HSC activation we observed in the 3D HepaRG/HSC model is not an artefact, we further characterized the system and test in the possible *in vivo* model. We based our additional characterization of the hepatic co-culture in 3 fibrosis features, observed *in vivo*:
- response to inflammation;
- inhibition of HSC activation by inhibiting HDAC; and
- fibrosis recovery

Following the key events of the fibrosis AOP, it is known that the recruitment of Macrophages and Kupffer cells will trigger the fibrotic response. Knowing that inflammation is one of the steps of APAP toxicity we mimicked the cytokine release from immune cells by performing the APAP exposure together with cytokine mixture (CK: TNFα, IL1β, IFNα and IFNγ). While CK alone (0 mM of APAP+CK) had no effect on the culture read-outs (Fig. 8A and B), when combined with the increasing concentrations of APAP, the toxic effect was potentiated (Fig. 8A), as well as the up-regulation of COL1A1, COL3A1 and LOXL2 (Fig. 8B). This assay proves the possibility to mimic the fibrotic inflammatory environment in the 3D co-culture. Furthermore, it also shows that there are stimuli that are not sufficient to induce a response of the system (CK alone has no effect on activation). Figure 3 A and B shows that HSC in the 3D HepaRG/HSC can activate as response to factors known to have a direct effect on these cells. For APAP, in order to confirm that the LOXL2, COL1A1 and COL1A3 up-regulations depicts HSC activation, we used valproic acid (VPA) an HDAC inhibitor, acting on HSC without changing hepatotoxicity, as previously shown by us (Mannaerts et al., 2010). In the study of Mannaerts et. al. VPA inhibited HSC activation both in 2D cultures and CCL₄ fibrosis mice, in the latter case, no decrease of the ALT values was observed. Identically, we observed that VPA did not affect APAP toxicity in the 3D HepaRG/HSC while we detect a lower up-regulation of the HSC markers at the highest APAP concentrations (Fig. 8 C and D). Finally, when we kept the culture in culture for additional 3 days upon APAP washout, we observed a decrease of the HSC activation markers (Fig. 8E) simulating HSC recover (Kisseleva, Cong et al. 2012).

### EXAMPLE 3: In vivo confirmation of the pro-fibrotic profile of the APAP

### MATERIAL AND METHODS

### Liver in vivo animal models

Male balbC and C57BL/6 mice were used for all experiments; animals were housed in a controlled environment with free access to chow and water. The experimental protocol was approved by the institutional Animal Care and Use Committee of Vrije Universiteit Brussel, permit number 14-212-2, and National Institutes of Health principles of laboratory animal care (NIH publication 86-23, revised 1995) were followed.

Acute and chronic models for liver fibrosis were used on mice of age 7-8 weeks. Acute injury was induced by a single dose of Acetaminophen (APAP) of 50; 150; 300 or 500mg APAP/kg weight. Chronic injury was induced by repeated injections of APAP twice per week for 4 weeks. Hepatic stellate cells were collected at the end of the treatment. Isolation of stellate cells was done using FACS, based on UV-positivity. After isolation cells were not cultured, but immediately used for mRNA analysis.

### RNA analysis

Total RNA was extracted from cells using the ReliaPrep RNA Cell Miniprep System (Promega, Madison, WI). Reverse-transcription using the Revert Aid Kit (ThermoFisher Scientific, St. Leon-Rot, Germany) allowed conversion of RNA to cDNA. The RT reaction was performed at 25 °C for ten minutes followed by 30 minutes at 50 °C. For quantitative real-time polymerase chain reaction (qPCR), GoTaq QPCR Master Mix with BRYTE green (Promega, Madison, WI) was used, subjected to qPCR in a 7500 real time PCR system and analysed using System SDS software v2.0.5 (Applied Biosystems), using GAPDH for normalisation. Reliability of GAPDH as house-keeping gene was determined using Normfinder (Andersen et al., 2004; Cancer Res 64(15): 5245-5250) to produce the ΔCT values. Primers were produced by Integrated DNA Technologies (Leuven, Belgium). Primers produced by Integrated DNA Technologies (Leuven, Belgium) are listed in Table 6.

**Table 6: Primers used for QPCR analysis**

| ***Gene*** | ***GenBank Acc N°*** | ***Forward Primer*** | ***SEQ ID N°*** | ***Reverse Primer*** | ***SEQ ID N°*** |
|---|---|---|---|---|---|
| Gapdh* | NM_008084 | TGTCCGTCGTGGATCTGA C | 33 | CCTGCTTCACCAC CTTCTTG | 37 |
| Acta 2* | NM_007392 | CCAGCACCATGAAGATCAAG | 34 | TGGAAGGTAGACAG CGAAGC | 38 |
| Col1a1* | NM_007742 | ACCTAAGGGTACCGCTGGA | 35 | TCCAGCTTCTCCATC TTTGC | 39 |
| Lox* | NM_010728 | CTCCTGGGAGTGGCACAG | 36 | CTTGCTTTGTGGCCT TCAG | 40 |

### RESULTS

In literature, as result of the most common liver injury observed for this compound, APAP is frequently classified as a hepatotoxic compound with no further pursue of other liver injury outcomes. Knowing that *in vivo,* HSC activate upon hepatocyte damage, especially when apoptosis and necrosis occurs, we decided to further investigate and at the same time validate our *in vitro* results (using the 3D hepatic co-culture) in terms of the pro-fibrotic capacities of this compound. As expected from the in vitro 3D hepatic co-culture results (see example 2), both upon acute and chronic exposure to doses equal or higher than 300 mg/kg weight we observed a significantly increase of the activation marker gene expression in the isolated HSC (Fig.9A), and even more pronounced in the chronic exposure (Fig. 9B). For the chronic exposure the fibrosis profile is confirmed at the protein level by the deposition of collagen around the portal area with septa formation, which is not observed in the liver of the control mice (data not shown). The increase of amount of cross-linked collagen upon APAP exposure is similar, but not at the same level, as CCL₄ mouse fibrosis model. These data validate the classification of APAP as a pro-fibrotic compound on top of its hepatotoxic classification.

### EXAMPLE 4: Testing of fibrosis-induced compounds

### Material and Methods

### Cell culture

Human Hepatic Stellate Cells (HSC) were isolated from hepatic non-parenchymal fraction and prepared for culture as described in Thoen *et al.,* 2011. Cells from two different donors were used, both male, healthy and under 13 years old. For the 3D cultures, cells were used between passages 5 and 10. Differentiated cryopreserved HepaRG® cells were obtained from Biopredic (Saint-Gregoire, France). On day 0 of culture, HSC were trypsinized and HepaRG thawn according to the instructions of the providers. For the generation of the cell spheroids, 96-well plates treated with cell- repellent coating (Greiner ref. 650970, Frickenhausen, Germany) were used. Mono- and co-culture suspensions were prepared and seeded at 10µl/well in the concentrations shown in table 7 below:

**Table 7: 3D cell seeding**

| *Type of culture* | *HSC seeding (x10⁵cells*/*ml)* | *HepaRG seeding (x10⁵cells*/*ml)* |
|---|---|---|
| 3D HepaRG | - | 2.00 |
| 3D HSC | 2.00 | - |
| 3D HepaRG/HSC | 1.33 | 0.67 |

After 15-30 min settling in the incubator, plates were placed on an orbital shaker, where stirring was kept at 80 rpm for the entire culture period. 24h after seeding, 100 µl of culture medium was added to each well and refreshed every second day (90%) thereafter. 3D HepaRG/HSC co-culture and control mono-cultures were cultured for 21 days. Cross diameter of the spheroids never exceeded 200 µm.

**Culture medium.** Day 0 cells were resuspended in HepaRG thawing medium and from day 1 to 21 cells were kept in the HepaRG culture medium 0% DMSO, until day 8. For the repeated versus single compound exposure assays, on day 8, the HepaRG culture medium 0% DMSO was replaced by the Serum-free HepaRG maintenance medium described in Klein et. *al.* 2013 (HepaRG Induction/Toxicity medium, DMSO 0.5% *(v*/*v),* 2 µg/ml of epidermal growth factor and 10 µg/ml of hepatocyte growth factor) and kept in this medium until the end of culture.

**Single and repeated exposure.** For the single and repeated compound exposure cell spheroids (3D HepaRG/HSC, 3D HepaRG and 3D HSC) were exposed respectively on day 20 for 24h or every second day from day 8 to day 21 in serum-free medium with no additional solvent. Cells were exposed to a range of 8 concentrations between 0.5 and 80mM for APAP single-dose, 0.125 and 40 mM for APAP repeated-dose, 0.3 and 500 µM Allyl Alcohol (AA, Sigma-Aldrich), 1.9 and 1000µM Methotrexate (MTX, Pfizer, NY, USA). For the recovery assay, on day 21, cells were washed using serum-free medium and kept for 3 extra days.

**Cell viability** assay was performed by measuring ATP production upon individual spheroid exposure to the different concentrations of the test compound. Cell spheroids were lysed individually for viability analysis using the Cell titer Glo kit (Promega) following the instructions of the manufacturer. Luminescence values were converted into percentage of ATP by comparison with controls and converted into dose-response curves using Prism 5 software.

### mRNA analysis

For the cell culture samples, total RNA from 4-6 pooled spheroids was extracted from cells using the RNeasy Micro Kit (Qiagen, Venlo, Limburg, Netherlands). In the mouse assays, sorted HSC from different mice were lysed separately. Total RNA from tissue cultured cells was extracted using ReliaPrep RNA Cell Miniprep System (Promega).
Reverse-transcription using the Revert Aid Kit (ThermoFisher Scientific, Waltham, Massachusetts, USA) allowed conversion of RNA to cDNA. The RT reaction was performed at 25 °C for ten minutes followed by 30 minutes at 50 °C. For quantitative real-time polymerase chain reaction (qPCR), GoTaq QPCR Master Mix with BRYTE green (Promega) was used, subjected to qPCR in a 7500 real time PCR system and analysed using System SDS software v2.0.5 (Applied Biosystems, Waltham, Massachusetts, USA), using GAPDH for normalisation. Reliability of GAPDH as house-keeping gene was determined using Normfinder (Anderson et al., 2004). Fold change differences between samples were determined using the comparative Ct (δδCt) method. The expression level of different targets, relative to GAPDH (δCt) and relative to the calibrator, was given by 2^{-δδCt}. Primers produced by Integrated DNA Technologies (Leuven, Belgium) are listed in Table 8.

**Table 8: Primers used for QPCR analysis (*mouse primers)**

| ***Gene*** | ***GenBank Acc N°*** | ***Forward Primer*** | ***SEQ ID N°*** | ***Reverse Primer*** | ***SEQ ID N°*** |
|---|---|---|---|---|---|
| GAPDH | NM_002046.4 | AGCCACATCGCTCAGAC AC | 1 | GCCCAATACGACCAAAT CC | 17 |
| COL1A1 | NM_000088.3 | GACACAGAGGTTTCAGT GG | 3 | CACCCTTAGCACCAACA G | 19 |
| COL3A1 | NM_000090.3 | GGAGCTGGCTACTTCTC GC | 10 | GGGAACATCCTCCTTCA ACAG | 26 |
| LOXL2 | NM_002318.2 | GGAGAGGACATACAATA CCAAAGTG | 11 | CCATGGAGAATGGCCAG TAG | 27 |

### Spheroid and tissue imaging.

**Paraffin sections.** 3D spheroids were fixed with 4 % (w/v) paraformaldehyde (PFA) with 4 % (w/v) sucrose in PBS for 20 min an embedded in paraffin as described in the work of Wong et. *al.* (Wong, Vosburgh et al. 2012). Mouse liver tissue from healthy and injured mice was formalin fixed and embedded in paraffin. After sectioned in 4-5 *µ*m-thick slices, samples were deparaffinazed and rehydrated in serial decreasing ethanol solutions before proceeding with the correspondent staining protocol.

**Immunohistochemistry.** Antigen retrieval citrate buffer (pH6.0) was used. This was followed by permeablization (PBS-Tween, 0.05%), blocked with 5% blocking solution (3% normal serum +2% BSA) and incubated with primary antibody. After rinsing, sections were incubated with streptavidin-peroxidase conjugate. Peroxidase reactivity was visualised with 3,3'-diaminobenzidine (DAB)/H2O2.

**Table 9: Antibodies used for Immunofluorescence and Immunohistochemistry**

| *Proteins* | *Supplier* | *Catn°* | *Application* |
|---|---|---|---|
| *CYP3A4* | *Cypex limited* | *PAP011* | *IF, 1*/*100* |
| *PDGFrβ* | *(APC labeled) abcam* | *ab119861* | *IF, 1*/*50* |
| *CASPASE 3* | *Cell Signaling Technology* | *9661* | *IHC, 1*/*100* |
| *COLLAGEN I* | *abeam* | *ab34710* | *IHC, 1*/*500* |
| *ACTA2* | *Sigma* | *A2547* | *IHC, 1*/*600* |

**Picrosirius Staining.** Slices were stained for 45 minutes with 0.1% Sirius Red phase green in a saturated picric acid solution. Picture quantification was carried out using ImageJ software.

### Statistics

Comparisons between more than two groups were analyzed for statistical significance by one-way ANOVA followed by Tukey. Statistical analysis of values for comparison between two groups was performed using an independent sample t-test. Results were considered significant when p <0.05.

### RESULTS

After testing and confirming the APAP effect in the indirect HSC activation described in the examples 2 and 3, we next finalized the characterization of the use of the co-culture in drug-induced fibrosis testing by exposing the cells to reference pro-fibrotic compounds. As such we used Allyl Alcohol (used to induce fibrosis in rat) and Methotrexate (classified as to induce fibrosis in some human subjects after prolonged treatment). Since the fibrotic effect of these compounds is observed upon long-term treatment, to cover any possible outcome, both compounds were tested in single and repeated fashion. Due to the kinetic interference of the serum in compound exposure, especially in repeated fashion we adapted the system to serum-free medium following the indications on the work of Klein *et al.* 2013. Before start we first ensured that at the end of the 21 days, hepatocyte gene expression was comparable with the previously used medium (data not shown). For HSC activation, APAP single and repeated was performed as positive control. We compared both single- and repeated-exposure (Table 9) by measuring cell viability and gene expression of the activation markers 24h upon last compound exposure.

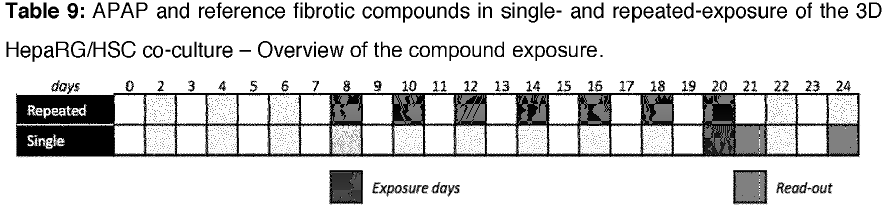

In the case of APAP, we observed toxicity and HSC activation both in single and repeated-exposure, however in the last one there is a shift of the peak effect to lower concentrations (Fig. 10 A, B) with an EC₅₀ shift from 10.4±2.3 to 2.25±0.35mM in average. The HSC activation follows the hepatotoxic shift with a peak of the repeated exposure at around 5-10 mM instead of 20-40mM (Fig. 10B). These results show the capacity of the cells to activate in the serum-free medium but also the increase of sensitivity upon the repeated exposure to the toxicant which we could also see by microscopic observation of the spheroids (data not shown).
For Methotrexate (MTX), single exposure does not seem to affect cell viability at any of the tested concentrations (Fig. 10C). However, upon repeated exposure, the percentage of ATP production even at the lowest concentration decreased to less than 20%. Nevertheless, even though the spheroids were smaller, the amount of disaggregating cells around the spheroid exposed repeatedly to 1.9 *µ*M MTX is less than the spheroid repeatedly exposed to 5 mM APAP, where the delimiting line of the sphere can no longer be observed (data not shown). In terms of HSC activation, the up-regulation of the markers upon 24h exposure to MTX, previously observed when cells were kept in serum containing medium (data not shown) was no longer observed (Fig. 10D). The incapacity of the cells to activate in the serum-free medium, was dramatically compensated once the 3D HepaRG/HSC culture was repeatedly exposed to the compound, for several concentrations.
Finally, for Allyl Alcohol, the dose-response observed at the single exposure disappeared upon repeated exposure, letting us to presume some toxicity resistance of the cells (Fig. 10E) explained by the metabolism of the compound (Covaci, Bucur et al. 2011). Nevertheless, both operational exposures lead to an increase of the HSC activation markers at mRNA levels, with a more consistent effect in the repeated-dose exposure (Fig. 10F). Repeated-dose exposures lead to a 100 fold increase of secreted collagen at day 14 (Fig. 10G) and a strong Sirius Red staining at the end of the experiment (Data not shown). Repeated exposure of MTX and Allyl alcohol to HepaRG/HSCs lead to high collagen secretion at day 14 which could not be observed at day 21, where the last 24h exposure lead to a higher or similar release of pro-collagen for the single exposure than after repeated MTX exposure (Fig. 10G). For both compounds, no collagen secretion is detected in the 3D mono-cultures (Fig. 10H).

Even if for APAP and MTX single exposure, the activation profile is similar to the 3D HepaRG, again, the basal levels are very different. Nevertheless, once in repeated-dose, the increase of the activation markers is higher, and sometimes unique for the 3D HepaRG/HSC (Fig. 11).

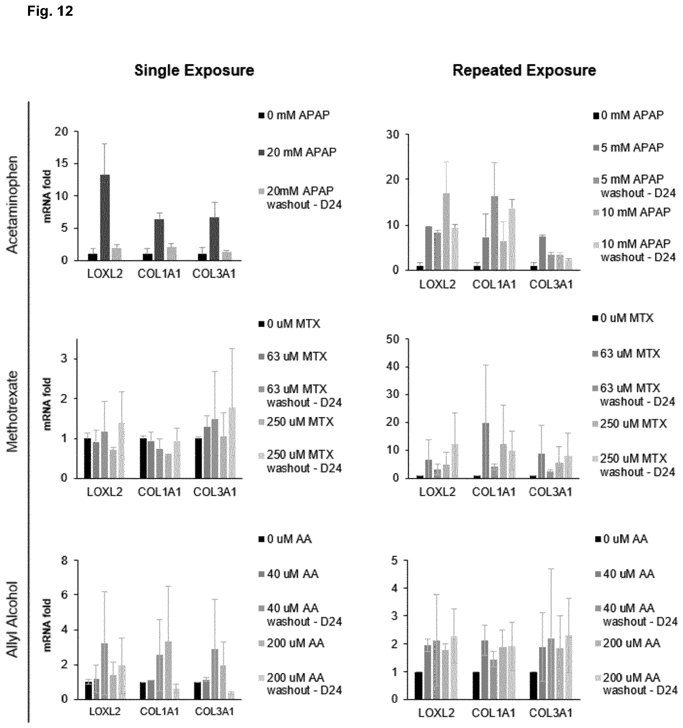

This assay shows that the 3D HepaRG/HSC co-culture has the capacity to respond positively to the reference fibrotic compounds and furthermore to have different responses depending on the nature of the compound. Once again, the capacity of cells to recover was checked 3 days after compound washout, however this was only observed in some cases such as APAP single exposure and MTX repeated exposure 63 *µ*M (Fig. 12) which again reflects the variability of the compound effect.

### GENERAL DISCUSSION

In conclusion, we can say that we have developed a human liver organoid with the main cellular players of fibrosis, in which drug-induced fibrosis can be reproduced. The system has the specificity that lacks in the hepatic mono-cultures and is sensitive to the different nature of the compounds, for toxicity, HSC activation and recovery. Taking into account that 40% of the drugs failing during clinical trials are due to DILI effects, the existence of a model like ours would represent a significant improvement in terms of costs, animal use and human predictability.

### REFERENCES

Andersen, C. L., J. L. Jensen and T. F. Orntoft (2004). "Normalization of real-time quantitative reverse transcription-PCR data: a model-based variance estimation approach to identify genes suited for normalization, applied to bladder and colon cancer data sets." Cancer Res 64(15): 5245-5250.
Covaci, O. I., B. Bucur and G. L. Radu (2011). "Acrolein detection based on alcohol dehydrogenase inhibition." International journal of Environmental Analytical Chemistry.
Kisseleva, T., M. Cong, Y. Paik, D. Scholten, C. Jiang, C. Benner, K. Iwaisako, T. Moore-Morris, B. Scott, H. Tsukamoto, S. M. Evans, W. Dillmann, C. K. Glass and D. A. Brenner (2012). "Myofibroblasts revert to an inactive phenotype during regression of liver fibrosis." Proc Natl Acad Sci U S A 109(24): 9448-9453.
Thoen, L. F., E. L. Guimaraes, L. Dolle, I. Mannaerts, M. Najimi, E. Sokal and L. A. van Grunsven (2011). "A role for autophagy during hepatic stellate cell activation." J Hepatol 55(6): 1353-1360.
Wong, C., E. Vosburgh, A. J. Levine, L. Cong and E. Y. Xu (2012). "Human neuroendocrine tumor cell lines as a three-dimensional model for the study of human neuroendocrine tumor therapy." J Vis Exp(66): e4218.
Lubberstedt, M. et al. "HepaRG human hepatic cell line utility as a surrogate for primary human hepatocytes in drug metabolism assessment in vitro." J Pharmacol Toxicol Methods 63, 59-68 (2011).
Mannaerts, I. et al. "Chronic administration of valproic acid inhibits activation of mouse hepatic stellate cells in vitro and in vivo." Hepatology 51, 603-614 (2010).
Watelet, J. et al. "Toxicity of chronic paracetamol ingestion." Aliment Pharmacol Ther 26, 1543-1544; author reply 1545-1546 (2007).

## Claims

1. A 3D co-culture comprising hepatocyte-like cells and hepatic stellate cells (HSCs); wherein said hepatic stellate cells are present in equal or excess amounts of said hepatocyte-like cells; and said cells are grown in direct contact with each other.

2. The 3D co-culture according to claim 1; wherein said co-culture is in the form of an organoid, more specifically a spheroid.

3. The 3D co-culture according to anyone of claims 1-2, wherein said hepatocyte-like cells are human hepatocyte-like cells; in particular human hepatocyte-like cells selected from the list comprising: primary human hepatocytes, HepaRG cells, human embryonic stem cells (hESC) differentiated into hepatocyte-like cells, human induced pluripotent stem cells (hiPSC) differentiated into hepatocyte-like cells, primary fibroblast transdifferentiated into hepatocyte-like cells, and hepatocyte-like cell lines such as HepG2 or Upcyted hepatocytes; more in particular HepaRG cells.

4. The 3D co-culture according to anyone of claims 1-2, wherein said hepatic stellate cells are human hepatic stellate cells; in particular human hepatic stellate cells selected from the list comprising: primary human hepatic stellate cells, human embryonic stem cells (hESC) differentiated into HSC-like cells, human induced pluripotent stem cells (hiPSC) differentiated into HSC-like cells, and human hepatic stellate cell lines such as LX-2, JS-1, hTERT-HSC, or upcyted-HSC; more in particular primary human hepatic stellate cells.

5. The 3D co-culture according to anyone of claims 1-4, wherein said 3D co-culture is free-floating in the culture medium.

6. The 3D co-culture according to anyone of claims 1-5, wherein the amount of HSC cells is about double the amount of hepatocyte-like cells.

7. Use of the 3D co-culture according to anyone of claims 1-6 in a screening assay for pro-fibrotic and/or anti-fibrotic compounds.

8. A screening assay for pro-fibrotic and/or anti-fibrotic compounds; said assay comprising the steps of:
(a) providing a 3D co-culture according to anyone of claims 1-7,
(b) incubating said co-culture with a test compound, and
(c) determining whether said compound induces or inhibits transdifferentiation and/or activation of said hepatic stellate cells;
wherein a compound that induces transdifferentiation and/or activation of said hepatic stellate cells is a pro-fibrotic compound; and wherein a compound that inhibits a fibrotic-compound induced-trans-differentiation and/or activation of said hepatic stellate cells is an anti-fibrotic compound.

9. The screening assay according to claim 8, wherein Col1a1, Col3a1 and/or Loxl2 expression levels are used as a read-out for hepatic stellate cell activation or transdifferentiation, in step (c).

10. The screening assay according to claim 9, wherein a compound that increases Col1a1, Col3a1 and/or Loxl2 expression is a pro-fibrotic compound; and wherein a compound that reduces Col1a1 and/or Loxl2 expression is an anti-fibrotic compound.

11. The screening assay according to anyone of claims 8 to 10, wherein said co-culture is incubated with said test compound as a single exposure or as repeated exposures.

## Patentansprüche

1. 3D-Co-Kultur, umfassend hepatozytenartige Zellen und hepatische Stellat-Zellen (HSCs), wobei die hepatischen Stellat-Zellen in gleichen oder überschüssigen Mengen der hepatozytenartigen Zellen vorliegen und die Zellen in direktem Kontakt miteinander gezüchtet werden.

2. 3D-Co-Kultur nach Patentanspruch 1, wobei die Co-Kultur in der Form eines Organoids, insbesondere eines Sphäroids, vorliegt.

3. 3D-Co-Kultur nach einem der Patentansprüche 1-2, wobei die hepatozytenartigen Zellen humane hepatozytenartige Zellen sind; insbesondere humane hepatozytenartige Zellen, ausgewählt aus der Liste umfassend: primäre humane Hepatozyten, HepaRG-Zellen, humane embryonale Stammzellen (hESC), die zu hepatozytenartigen Zellen differenziert sind, humane induzierte pluripotente Stammzellen (hiPSC), die zu hepatozytenartigen Zellen differenziert sind, primäre Fibroblasten, die zu hepatozytenartigen Zellen transdifferenziert sind, und hepatozytenartige Zelllinien wie HepG2 oder Upcyted Hepatozyten; insbesondere HepaRG-Zellen.

4. 3D-Co-Kultur nach einem der Patentansprüche 1-2, wobei die hepatischen Stellat-Zellen humane hepatische Stellat-Zellen sind; insbesondere humane hepatische Stellat-Zellen, ausgewählt aus der Liste, umfassend: primäre humane hepatische Stellat-Zellen, humane embryonale Stammzellen (hESC), die zu HSC-ähnlichen Zellen differenziert sind, humane induzierte pluripotente Stammzellen (hiPSC), die zu HSC-ähnlichen Zellen differenziert sind, und humane hepatische Stellat-Zelllinien wie LX-2, JS-1, hTERT-HSC oder upcyted-HSC; insbesondere primäre humane hepatische Stellat-Zellen.

5. 3D-Co-Kultur nach einem der Patentansprüche 1-4, wobei die 3D-Co-Kultur im Kulturmedium freischwebend ist.

6. 3D-Co-Kultur nach einem der Patentansprüche 1-5, wobei die Menge an HSC-Zellen etwa doppelt so groß ist wie die Menge an hepatozytenartigen Zellen.

7. Verwenden der 3D-Co-Kultur nach einem der vorhergehenden Patentansprüche 1-6 in einem Screening-Assay für pro-fibrotische und/oder anti-fibrotische Verbindungen.

8. Screening-Assay für pro-fibrotische und/oder anti-fibrotische Verbindungen; wobei das Assay die folgenden Schritte umfasst:
(a) Bereitstellen einer 3D-Co-Kultur nach einem der vorhergehenden Patentansprüche 1-7,
(b) Inkubieren der Co-Kultur mit einer Testverbindung, und
(c) Ermitteln, ob die Verbindung die Transdifferenzierung und/oder Aktivierung der hepatischen Stellat-Zellen induziert oder hemmt;
wobei eine Verbindung, die eine Transdifferenzierung und/oder Aktivierung der hepatischen Stellat-Zellen induziert, eine pro-fibrotische Verbindung ist; und wobei eine Verbindung, die eine durch eine fibrotische Verbindung induzierte Transdifferenzierung und/oder Aktivierung der hepatischen Stellat-Zellen hemmt, eine anti-fibrotische Verbindung ist.

9. Screening-Assay nach Patentanspruch 8, wobei Col1a1-, Col3a1- und/oder Loxl2-Expressionsniveaus in Schritt (c) als Messwert für die Aktivierung oder Transdifferenzierung hepatischer Stellat-Zellen verwendet werden.

10. Screening-Assay nach Patentanspruch 9, wobei eine Verbindung, die die Col1a1-, Col3a1- und/oder Loxl2-Expression erhöht, eine pro-fibrotische Verbindung ist; und wobei eine Verbindung, die die Col1a1- und/oder Loxl2-Expression reduziert, eine anti-fibrotische Verbindung ist.

11. Screening-Assay nach einem der Patentansprüche 8 bis 10, wobei die Co-Kultur mit der Testverbindung als einmalige Exposition oder als wiederholte Exposition inkubiert wird.

## Revendications

1. Co-culture 3D comprenant des cellules de type hépatocyte et des cellules stellaires hépatiques (CSHs) ; dans laquelle lesdites cellules stellaires hépatiques sont présentes en quantités égales ou en excès desdites cellules de type hépatocyte ; et lesdites cellules sont cultivées en contact direct les unes avec les autres.

2. Co-culture 3D selon la revendication 1 ; dans laquelle ladite co-culture est sous la forme d'un organoïde, plus précisément un sphéroïde.

3. Co-culture 3D selon l'une quelconque des revendications 1 à 2, dans laquelle lesdites cellules de type hépatocyte sont des cellules de type hépatocyte humain ; en particulier des cellules de type hépatocyte humain sélectionnées dans la liste comprenant : les hépatocytes humains primaires, les cellules HepaRG, les cellules souches embryonnaires humaines (CSEh) différenciées en cellules de type hépatocyte, les cellules souches pluripotentes induites humaines (CSPih) différenciées en cellules de type hépatocyte, les fibroblastes primaires transdifférenciés en cellules de type hépatocyte, et les lignées cellulaires de type hépatocyte telles que les hépatocytes HepG2 ou Upcyte ; plus particulièrement les cellules HepaRG.

4. Co-culture 3D selon l'une quelconque des revendications 1 à 2, dans laquelle lesdites cellules stellaires hépatiques sont des cellules stellaires hépatiques humaines ; en particulier des cellules stellaires hépatiques humaines sélectionnées dans la liste comprenant : les cellules stellaires hépatiques humaines primaires, les cellules souches embryonnaires humaines (CSEh) différenciées en cellules de type HSC, les cellules souches pluripotentes induites humaines (CSPih) différenciées en cellules de type HSC, et les lignées de cellules stellaires hépatiques humaines telles que LX-2, JS-1, hTERT-HSC, ou HSC upcyte ; plus particulièrement les cellules stellaires hépatiques humaines primaires.

5. Co-culture 3D selon l'une quelconque des revendications 1 à 4, dans laquelle ladite co-culture 3D flotte librement dans le milieu de culture.

6. Co-culture 3D selon l'une quelconque des revendications 1 à 5, dans laquelle la quantité de cellules HSC est environ le double de la quantité de cellules de type hépatocyte.

7. Utilisation de la co-culture 3D selon l'une quelconque des revendications 1 à 6 dans un essai de criblage destiné à des composés profibrotiques et/ou anti-fibrotiques.

8. Essai de criblage destiné aux composés profibrotiques et/ou anti-fibrotiques ; ledit essai comprenant les étapes suivantes :
(a) la fourniture d'une co-culture 3D selon l'une quelconque des revendications 1 à 7,
(b) l'incubation de ladite co-culture avec un composé test, et
(c) la détermination du fait que ledit composé induit ou inhibe la transdifférenciation et/ou l'activation desdites cellules stellaires hépatiques ;
dans lequel un composé qui induit une transdifférenciation et/ou une activation desdites cellules stellaires hépatiques est un composé profibrotique ; et dans lequel un composé qui inhibe une transdifférenciation et/ou une activation induite par un composé fibrotique desdites cellules stellaires hépatiques est un composé anti-fibrotique.

9. Essai de criblage selon la revendication 8, dans lequel les niveaux d'expression de Col1a1, Col3a1 et/ou Loxl2 sont utilisés comme une lecture destinée à l'activation ou la transdifférenciation des cellules stellaires hépatiques, dans l'étape (c).

10. Essai de criblage selon la revendication 9, dans lequel un composé qui augmente l'expression de Col1a1, Col3a1 et/ou Loxl2 est un composé profibrotique ; et dans lequel un composé qui réduit l'expression de Col1a1 et/ou Loxl2 est un composé anti-fibrotique.

11. Essai de criblage selon l'une quelconque des revendications 8 à 10, dans lequel ladite co-culture est incubée avec ledit composé test en tant qu'exposition unique ou en tant qu'expositions répétées.


### SEQUENCE LISTING

<110> Vrije Universiteit Brussel
<120> HUMAN HEPATIC 3D CO-CULTURE MODEL AND USES THEREOF
<130> VUB-002
<150> GB1505012.3
   <151> 2015-03-25
<150> GB1410048.1
   <151> 2014-06-06
<160> 40
<170> BiSSAP 1.3
<210> 1
   <211> 19
   <212> DNA
   <213> Artificial Sequence
<220>
   <223> Primer
<400> 1
   agccacatcg ctcagacac 19
<210> 2
   <211> 20
   <212> DNA
   <213> Artificial Sequence
<220>
   <223> Primer
<400> 2
   ctgttccagc catccttcat 20
<210> 3
   <211> 19
   <212> DNA
   <213> Artificial Sequence
<220>
   <223> Primer
<400> 3
   gacacagagg tttcagtgg 19
<210> 4
   <211> 22
   <212> DNA
   <213> Artificial Sequence
<220>
   <223> Primer
<400> 4
   cacagttgtc atcaacatta cc 22
<210> 5
   <211> 24
   <212> DNA
   <213> Artificial Sequence
<220>
   <223> Primer
<400> 5
   gatgagtaca aaagtcctga tcca 24
<210> 6
   <211> 26
   <212> DNA
   <213> Artificial Sequence
<220>
   <223> Primer
<400> 6
   gctaaccttt gatgctataa ctacga 26
<210> 7
   <211> 18
   <212> DNA
   <213> Artificial Sequence
<220>
   <223> Primer
<400> 7
   agtctctgcc gcccttct 18
<210> 8
   <211> 22
   <212> DNA
   <213> Artificial Sequence
<220>
   <223> Primer
<400> 8
   cctctctcta atcagccctc tg 22
<210> 9
   <211> 20
   <212> DNA
   <213> Artificial Sequence
<220>
   <223> Primer
<400> 9
   agctacgaat ctccgaccac 20
<210> 10
<211> 19
   <212> DNA
   <213> Artificial Sequence
<220>
   <223> Primer
<400> 10
   ggagctggct acttctcgc 19
<210> 11
   <211> 25
   <212> DNA
   <213> Artificial Sequence
<220>
   <223> Primer
<400> 11
   ggagaggaca tacaatacca aagtg 25
<210> 12
   <211> 22
   <212> DNA
   <213> Artificial Sequence
<220>
   <223> Primer
<400> 12
   acgcctggtt actatcaaaa gg 22
<210> 13
   <211> 20
   <212> DNA
   <213> Artificial Sequence
<220>
   <223> Primer
<400> 13
   tggcacaatg aagtgggtaa 20
<210> 14
   <211> 19
   <212> DNA
   <213> Artificial Sequence
<220>
   <223> Primer
<400> 14
   cctccctgaa agattcagc 19
<210> 15
   <211> 19
   <212> DNA
   <213> Artificial Sequence
<220>
   <223> Primer
<400> 15
   ctgcccgtat gtccacctg 19
<210> 16
   <211> 20
   <212> DNA
   <213> Artificial Sequence
<220>
   <223> Primer
<400> 16
   gcaatagcaa tgattggtcc 20
<210> 17
   <211> 19
   <212> DNA
   <213> Artificial Sequence
<220>
   <223> Primer
<400> 17
   gcccaatacg accaaatcc 19
<210> 18
   <211> 21
   <212> DNA
   <213> Artificial Sequence
<220>
   <223> Primer
<400> 18
   tcatgatgct gttgtaggtg g 21
<210> 19
   <211> 18
   <212> DNA
   <213> Artificial Sequence
<220>
   <223> Primer
<400> 19
   cacccttagc accaacag 18
<210> 20
   <211> 25
   <212> DNA
   <213> Artificial Sequence
<220>
   <223> Primer
<400> 20
   ctgaccttta ggatatagtt tccag 25
<210> 21
   <211> 19
   <212> DNA
   <213> Artificial Sequence
<220>
   <223> Primer
<400> 21
   ctgcagccac tggttctgt 19
<210> 22
   <211> 21
   <212> DNA
   <213> Artificial Sequence
<220>
   <223> Primer
<400> 22
   tttgtgcgca tgtagaatct g 21
<210> 23
   <211> 19
   <212> DNA
   <213> Artificial Sequence
<220>
   <223> Primer
<400> 23
   gtgactgggg cattgattg 19
<210> 24
   <211> 21
   <212> DNA
   <213> Artificial Sequence
<220>
   <223> Primer
<400> 24
   gaggacctgg gagtagatga g 21
<210> 25
   <211> 22
   <212> DNA
   <213> Artificial Sequence
<220>
   <223> Primer
<400> 25
   cgttatccca tgtgtcgaag aa 22
<210> 26
   <211> 21
   <212> DNA
   <213> Artificial Sequence
<220>
   <223> Primer
<400> 26
   gggaacatcc tccttcaaca g 21
<210> 27
   <211> 20
   <212> DNA
   <213> Artificial Sequence
<220>
   <223> Primer
<400> 27
   ccatggagaa tggccagtag 20
<210> 28
   <211> 23
   <212> DNA
   <213> Artificial Sequence
<220>
   <223> Primer
<400> 28
   cagacccatt tacttgtgtt gga 23
<210> 29
   <211> 20
   <212> DNA
   <213> Artificial Sequence
<220>
   <223> Primer
<400> 29
   ctgagcaaag gcaatcaaca 20
<210> 30
   <211> 20
   <212> DNA
   <213> Artificial Sequence
<220>
   <223> Primer
<400> 30
   tgaagaagtc ctcctaagct 20
<210> 31
<211> 21
   <212> DNA
   <213> Artificial Sequence
<220>
   <223> Primer
<400> 31
   agctcctcga cgtagtagag a 21
<210> 32
   <211> 19
   <212> DNA
   <213> Artificial Sequence
<220>
   <223> Primer
<400> 32
   ccaacccatc gagaatcag 19
<210> 33
   <211> 19
   <212> DNA
   <213> Artificial Sequence
<220>
   <223> Primer
<400> 33
   tgtccgtcgt ggatctgac 19
<210> 34
   <211> 20
   <212> DNA
   <213> Artificial Sequence
<220>
   <223> Primer
<400> 34
   ccagcaccat gaagatcaag 20
<210> 35
   <211> 19
   <212> DNA
   <213> Artificial Sequence
<220>
   <223> Primer
<400> 35
   acctaagggt accgctgga 19
<210> 36
   <211> 18
   <212> DNA
   <213> Artificial Sequence
<220>
   <223> Primer
<400> 36
   ctcctgggag tggcacag 18
<210> 37
   <211> 20
   <212> DNA
   <213> Artificial Sequence
<220>
   <223> Primer
<400> 37
   cctgcttcac caccttcttg 20
<210> 38
   <211> 20
   <212> DNA
   <213> Artificial Sequence
<220>
   <223> Primer
<400> 38
   tggaaggtag acagcgaagc 20
<210> 39
   <211> 20
   <212> DNA
   <213> Artificial Sequence
<220>
   <223> Primer
<400> 39
   tccagcttct ccatctttgc 20
<210> 40
   <211> 19
   <212> DNA
   <213> Artificial Sequence
<220>
   <223> Primer
<400> 40
   cttgctttgt ggccttcag 19